# EUROPÄISCHE PATENTSCHRIFT

(11) **EP 0 647 631 B1**
(45) Veröffentlichungstag und Bekanntmachung des Hinweises auf die Patenterteilung: **16.08.2001**
(21) Anmeldenummer: 94115579.8
(22) Anmeldetag: 04.10.1994
(51) Int. Cl.: C07D 239/34, C07D 239/52, A01N 43/54

(54) **Naphthylether, Verfahren zu ihrer Verwendung, sie enthaltende Mittel und ihre Verwendung als Fungizide**
Naphthylether, processes for their use, agents containing them and their use as fungicides
Ethers naphtylique, procédés pour leurs utilisations, agents les contenant et leur utilisation comme fongicide

(30) Priorität: 12.10.1993 DE 4334709
(43) Veröffentlichungstag der Anmeldung: 12.04.1995
(73) Patentinhaber: BASF Aktiengesellschaft, 67063 Ludwigshafen (DE)
(72) Erfinder: Grammenos, Wassilios, Dr., D-67063 Ludwigshafen (DE); Kirstgen, Reinhard, Dr., D-67434 Neustadt (DE); König, Hartmann, Dr., D-67117 Limburgerhof (DE); Oberdorf, Klaus, Dr., D-69117 Heidelberg (DE); Sauter, Hubert, Dr., D-68167 Mannheim (DE); Lorenz, Gisela, Dr., D-67434 Neustadt (DE); Ammermann, Eberhard, Dr., D-64646 Heppenheim (DE)

(56) Entgegenhaltungen:
- EP-A- 0 253 213
- EP-A- 0 254 426
- EP-A- 0 374 811
- EP-A- 0 385 357
- EP-A- 0 468 684
- EP-A- 0 513 580
- EP-A- 0 546 387

## Beschreibung

Die vorliegende Erfindung betrifft Naphthylether der allgemeinen Formel I 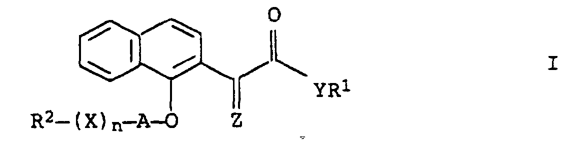 in der der Index und die Substituenten die folgende Bedeutung haben:
- A: eine der Gruppen A.1 bis A.42 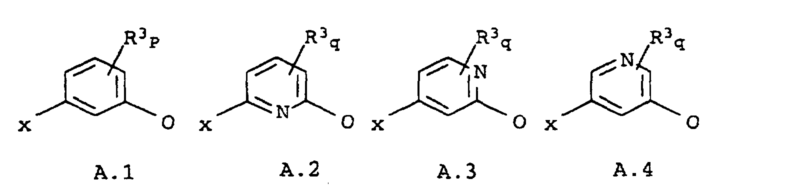 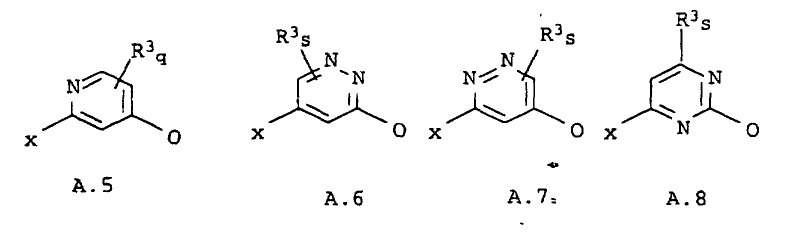 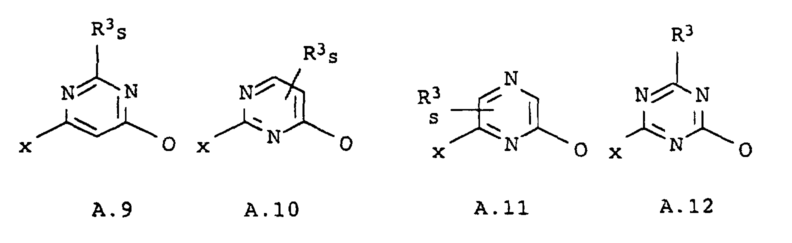 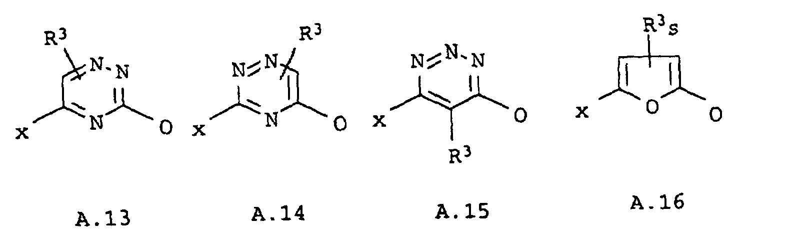 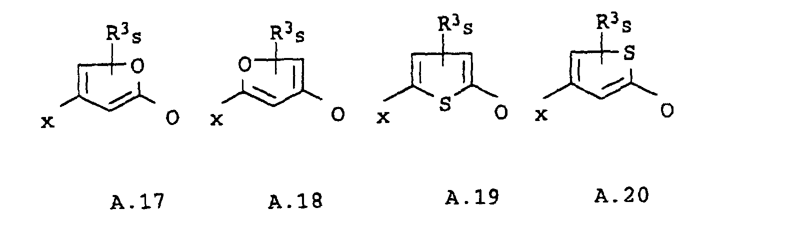 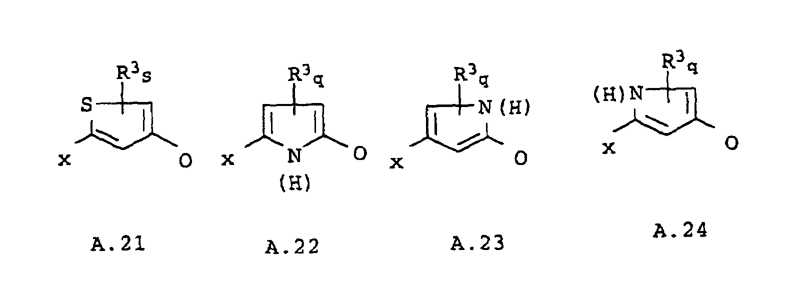 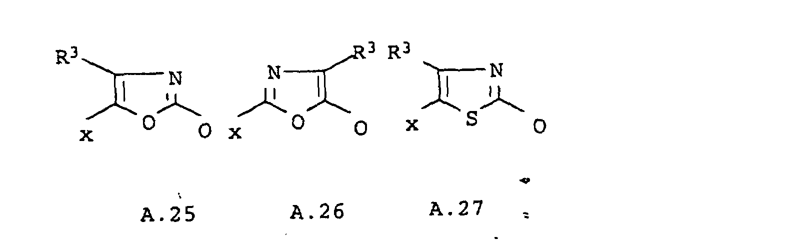 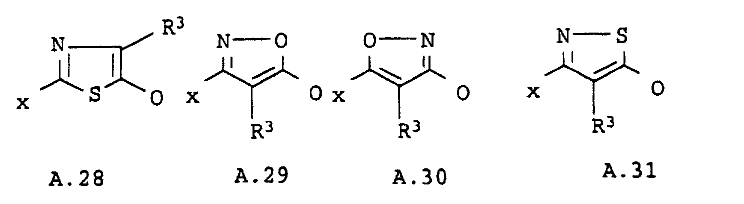 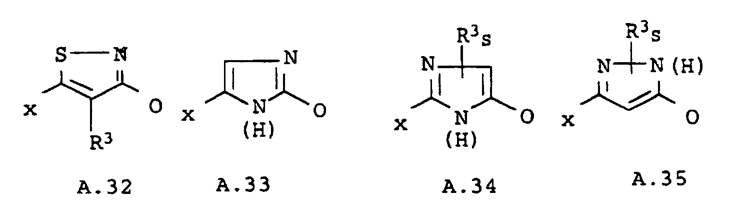 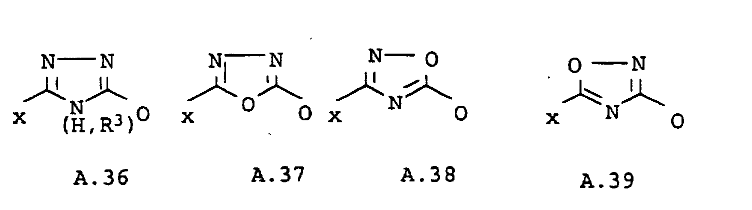 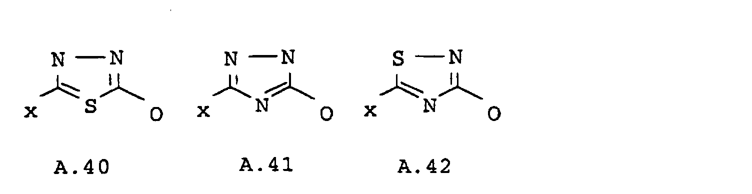
worin die Bezeichnungen x- und -o dazu dienen anzugeben, zu welchem der A benachbarten Reste die Bindung gerichtet ist, nämlich die mit x- Bezeichnete Bindung zum Rest -(X)ₙR² und die mit -o bezeichnete Bindung zum Sauerstoffatom, und worin
- p: 0, 1, 2, 3 oder 4 bedeutet, wobei die Reste R³ verschieden sein können, wenn p > 1 ist;
- q: 0, 1, 2 oder 3 bedeutet, wobei die Reste R³ verschieden sein können, wenn q > 1 ist;
- s: 0, 1 oder 2 bedeutet, wobei die Reste R³ verschieden sein können, wenn s > 1 ist;
wobei die Reste R³ an ein C-Atom des Rings oder, im Fall der Reste A.22, A.23, A.24, A.33, A.34, A.35 und A.36 zusätzlich auch an ein N-Atom des Rings gebunden sind und
- R³: die folgende Bedeutung hat:
Wasserstoff, Halogen, C₁-C₄-Alkyl, C₁-C₄-Halogenalkyl, C₁-C₄- Halogenalkoxy und C₃-C₆-Cycloalkyl;
- n: 0 oder 1;
- X: Sauerstoff, Schwefel oder Stickstoff, wobei das Stickstoffatom einen der folgenden Reste trägt: Wasserstoff, C₁-C₄-Alkyl, C₁-C₄-Alkoxy, C₃-C₆-Cycloalkyl, Phenyl oder Benzyl, wobei der Phenylring und der Benzylrest ein bis fünf Halogenatome oder ein bis drei der folgenden Reste tragen können: Halogen C₁-C₄-Alkyl, C₁-C₄-Halogenalkyl, C₁-C₄-Alkoxy, C₁-C₄- Halogenalkoxy, C₃-C₆-Cycloalkyl, Phenyl oder Benzyl;
- Y: Sauerstoff, eine direkte Bindung oder
für den Fall, daß Z für NOCH₃ steht: Sauerstoff, eine direkte Bindung oder Stickstoff, wobei das Stickstoffatom einen der folgenden Reste trägt: Wasserstoff, C₁-C₄-Alkyl oder C₁-C₄- Alkoxy;
- Z: CHOCH₃, NOCH₃, CHCH₃ oder CHCH₂CH₃;
- R¹: C₁-C₄-Alkyl
- R²: C₁-C₆-Alkyl, C₃-C₆-Cycloalkyl, C₂-C₆-Alkenyl oder C₃-C₆-Alkinyl, wobei diese Gruppen partiell oder vollständig halogeniert sein können und/oder einen oder zwei der folgenden Reste tragen können:
Hydroxy, Cyano, Isocyano, Nitro, Formyl, Amino, Formylamino, Carboxyl, Sulfoxyl, Carbonylamino, C₁-C₈-Alkylamino, Di-C₁-C₈-alkylamino, C₁-C₈-Alkylcarbonyl, C₁-C₈-Alkoxycarbonyl, C₁-C₈-Alkylaminocarbonyl, C₁-C₈-Alkylsulfonyl oder Phenylsulfonyl;
Phenyl, welches ein bis fünf Halogenatome und/oder ein bis drei der folgenden Reste tragen kann:
Amino, Carboxyl, Carbonylamino, Cyano, Formyl, Formylamino, Hydroxy, Isocyano, Nitro, Sulfoxyl, Thiocarboxamido, Thiocyanato, C₁-C₈-Alkyl, C₁-C₄-Halogenalkyl, C₁-C₈-Alkylcarbonyl, C₁-C₈-Alkylcarbonyloxy, C₁-C₈-Alkylcarbonylthio, C₁-C₈-Alkylcarbonylamino, C₁-C₈-Alkylsulfonyl, C₁-C₈-Alkylsulfonyloxy, C₁-C₈-Alkylsulfonylamino, C₁-C₈-Alkoxy, C₁-C₄-Halogenalkoxy, C₁-C₈-Alkoxycarbonyl, C₁-C₆-Alkoxyiminoalkyl, C₁-C₈-Alkylthio, C₁-C₈-Alkylthiocarbonyl, C₁-C₈-Alkylamino, C₁-C₈-Alkylaminocarbonyl, Di-C₁-C₈-alkylamino, Di-C₁-C₈-alkylaminocarbonyl, C₃-C₈-Cycloalkyl, C₃-C₈-Cycloalkoxy, C₃-C₈-Cycloalkylthio, C₃-C₈-Cycloalkylamino, C₂-C₁₀-Alkenyl, C₂-C₈-Alkenyloxy, C₂-C₁₀-Alkinyl, C₂-C₁₀-Alkinyloxy, Phenylsulfonyl, Phenylsulfonyloxy oder Phenylsulfonylamino;
Furyl, Thienyl, Pyrrolyl, Pyrazolyl, Imidazolyl, Triazolyl, Isoxazolyl, Oxazolyl, Oxadiazolyl, Isothiazolyl, Thiazolyl, Thiadiazolyl, Pyridyl, Pyridazinyl, Pyrimidyl, Pyrazinyl oder Triazinyl, wobei diese Ringe ein bis vier Halogenatome und/oder ein bis drei der folgenden Reste tragen können:
Amino, Carboxyl, Carbonylamino, Cyano, Formyl, Formylamino, Hydroxy, Isocyano, Nitro, Sulfoxyl, Thiocarboxamido, Thiocyanato, C₁-C₈-Alkyl, C₁-C₄-Halogenalkyl, C₁-C₈-Alkylcarbonyl, C₁-C₈-Alkylcarbonyloxy, C₁-C₈-Alkylcarbonylthio, C₁-C₈-Alkylcarbonylamino, C₁-C₈-Alkylsulfonyl, C₁-C₈-Alkylsulfonyloxy, C₁-C₈-Alkylsulfonylamino, C₁-C₈-Alkoxy, C₁-C₄-Halogenalkoxy, C₁-C₈-Alkoxycarbonyl, C₁-C₆-Alkoxyiminoalkyl, C₁-C₈-Alkylthio, C₁-C₈-Alkylthiocarbonyl, C₁-C₈-Alkylamino, C₁-C₈-Alkylaminocarbonyl, Di-C₁-C₈-alkylamino, Di-C₁-C₈-alkylaminocarbonyl, C₃-C₈-Cycloalkyl, C₃-C₈-Cycloalkoxy, C₃-C₈-Cycloalkylthio, C₃-C₈-Cycloalkylamino, C₂-C₁₀-Alkenyl, C₂-C₈-Alkenyloxy, C₂-C₁₀-Alkinyl, C₂-C₁₀-Alkinyloxy, Phenylsulfonyl, Phenylsulfonyloxy oder Phenylsulfonylamino;
oder, für den Fall, daß der Wert von n 0 beträgt, bedeutet R² zusätzlich zu den vorstehenden Bedeutungen: Halogen, C₁-C₆-Alkoxy, C₁-C₄-Alkylsulfoxyl oder Phenylsulfoxyl, wobei der Phenylring seinerseits ein bis fünf Halogenatome und/oder ein bis drei der folgenden Substituenten tragen kann: Halogen, C₁-C₄-Alkyl, C₁-C₄-Halogenalkyl, C₁-C₄-Alkoxy, C₁-C₄-Halogenalkoxy, C₃-C₆-Cycloalkyl, Phenyl oder Benzyl.

Aus der Literatur (EP-A 385 357) sind Naphthylether mit Aryloxy-, Arylthio- oder Arylalkoxyalkyletherfunktion als Fungizide bekannt.

Aufgabe der vorliegenden Erfindung waren neue Naphthylether mit verbessertem und verbreitertem Wirkungsspektrum.

Demgemäß wurden die eingangs definierten Naphthylether I gefunden. Außerdem wurden Verfahren zur Herstellung der Naphthylether I, sie enthaltende Mittel und ihre Verwendung zur Bekämpfung tierischer Schädlinge oder Schadpilze gefunden.

Die erfindungsgemäßen Verbindungen sind auf verschiedenen, in analoger Weise aus der Literatur bekannten, Wegen zugänglich. Ausgehend von 1-Naphthol (II) erhält man die Verbindungen I, in denen Y für Sauerstoff steht, bevorzugt nach einem der im folgenden Reaktionsschema zusammengestellten Verfahren:

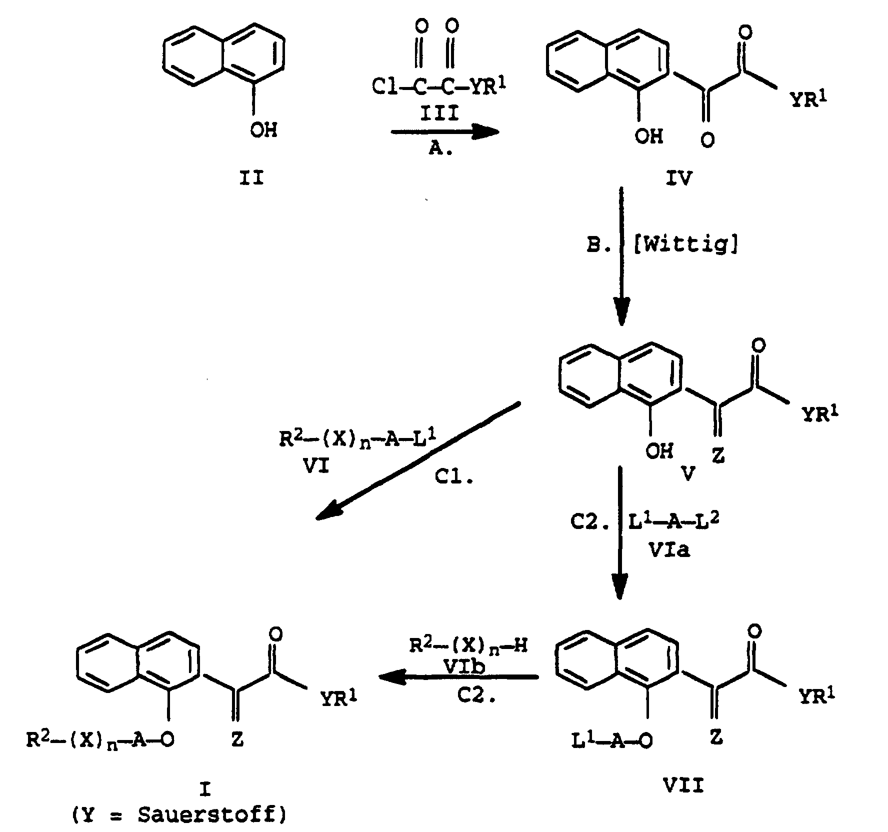

Die Synthese der Verbindungen I, in denen Y für Sauerstoff steht, umfaßt jeweils
A) die Acylierung der 2-Position des Naphthylgerüsts (Umsetzung von II mit III);
B) Einführung der Gruppe Z (Umsetzung von IV zu V); durch
   1. eine Wittig- oder Wittig-analoge Reaktion der alpha-Ketocarbonylgruppe für Verbindungen I, in denen Z für CHOCH₃, CHCH₃ oder CHCH₂CH₃ steht oder
   2. Umsetzung mit O-Methylhydroxylamin oder mit Hydroxylamin und anschließender Methylierung für Verbindungen I, in denen Z für NOCH₃ steht;
C1) die Veretherung des Naphthols in einer Stufe (Umsetzung von V zu I oder
C2) die Veretherung des Naphthols in zwei Stufen (Umsetzung von V über VII zu I).

Die entsprechenden Verbindungen I, in denen Y für ein ggf. subst. Stickstoffatom steht, erhält man durch Umsetzung von Verbindungen V, VII oder I, in denen Y für Sauerstoff steht, mit einem entsprechenden Amin.

Die Umsetzungen werden im einzelnen üblicherweise wie folgt durchgeführt. In den folgenden Reaktionsgleichungen sind die nicht an der betreffenden Umsetzung beteiligten Gruppen jeweils durch -# (für die 2-Position des Naphthylrings) bzw. -O* (für die 1-Position des Naphthylrings) abgekürzt.
A) Die Acylierung der 2-Position des Naphthylgerüsts (Umsetzung von II mit III) 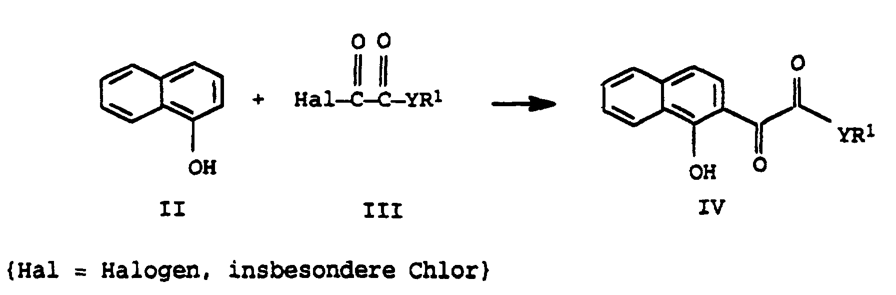
   Die Acylierung des Naphthols II erfolgt durch Umsetzung mit Oxalsäuremethylesterhalogeniden, insbesondere dem Chlorid, in an sich bekannter Weise (Piccolo et al., Tetrahedron 42, 885 (1986); EP-A 385 357) in einem inerten organischen Lösungsmittel (z.B. Dichlorethan) in Gegenwart einer Lewis-Säure (z.B. Titantetrachlorid).
B.1 Einführung der Gruppe Z (Umsetzung von IV zu V) durch eine Wittig- oder Wittig-analoge Reaktion der alpha-Ketocarbonylgruppe für Verbindungen I, in denen Z für CHOCH₃, CHCH₃ oder CHCH₂CH₃ steht 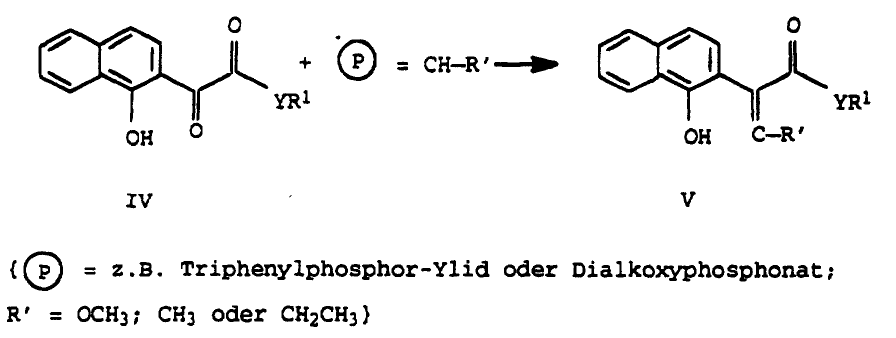
   Die Umsetzung der Verbindungen IV zu den entsprechenden Derivaten V I erfolgt durch Wittig- oder Wittig-analoge Reaktion in an sich bekannter Weise (EP-A 44 448; EP-A 385,357).
B.2 Einführung der Gruppe Z (Umsetzung von IV zu V) durch Umsetzung mit O-Methylhydroxylamin oder mit Hydroxylamin und anschließender Methylierung für Verbindungen I, in denen Z für NOCH₃ steht 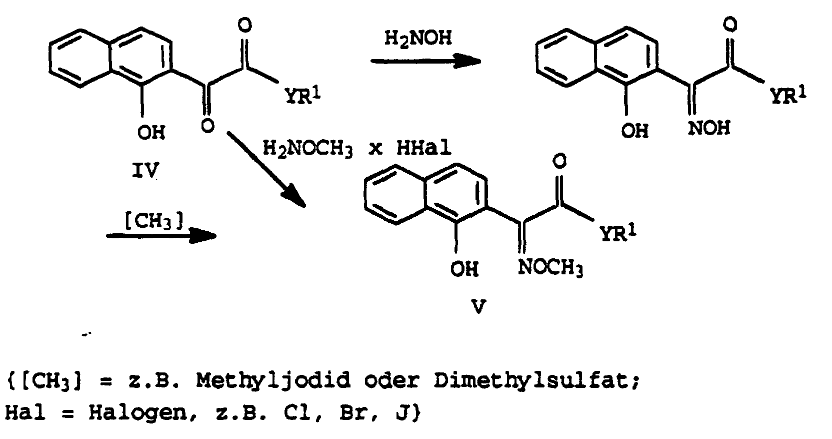
   Die Einführung der Gruppe Z = NOCH₃ erfolgt durch Umsetzung mit O-Methylhydroxylamin oder durch Umsetzung mit Hydroxylamin zum entsprechenden Oxim und anschließender Methylierung in an sich bekannter Weise (EP-A 253 213; EP-A 385 357).
C1) Die Veretherung des Naphthols in einer Stufe (Umsetzung von V zu I) oder 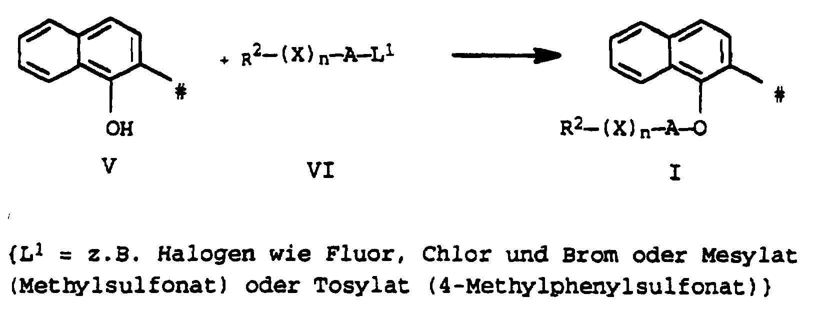
   Diese Umsetzung wird üblicherweise bei Temperaturen von 0°C bis 120°C, vorzugsweise 20°C bis 80°C durchgeführt.
   Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, Ketone wie Aceton und Methylethylketon .sowie Dimethylsulfoxid, Dimethylformamid, Dimethylacetamid, 1,3-Dimethylimidazolidin-2-on und 1,3-Dimethyltetrahydro-2(1H)-pyrimidin besonders bevorzugt Acetonitril, Methylenchlorid, Aceton und Dimethylformamid. Es können auch Gemisch der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydrine wie Lithiumhydrid, Natriumhydrid, Kaliumhydrid und Calziumhydrid, Alkalimetall- und Erdalkalimetallcarbonate wie Kaliumcarbonate und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriummethanolat, Kaliummethanolat, Kalium- tert.-butanolat und Dimethoxymagnesium außerdem organische Basen, z. B. tertiare Amine wie Trimethylamin, Triethylamin, Di-isopropylethylamin und N-Methylpiperidin, Pyridin, substituierte Pyridine wie Collidin, Lutidin und 4-Dimethylaminopyridin sowie icyclische Amine in Betracht.
   Besonders bevorzugt werden Natriumhydroxid, Natriumhydrid, Kaliumcarbonat und Kalium-tert.-butanolat.
   Die Basen werden im allgemeinen äquimolar, im Überschuß oder gegebenenfalls als Lösungsmittel verwendet.
   Es kann für die Umsetzung vorteilhaft sein, eine katalytische Menge eines Kronenethers wie z. B. 18-Krone-6 oder 15-Krone-5 zuzusetzen.
   Die Umsetzung kann auch in Zweiphasensystemen bestehen aus einer Lösung von Alkali- oder Erdalkalihydroxiden oder Alkali- oder Erdalkalicarbonaten in Wasser und einer organischen Phase wie z. B. halogenierten Kohlenwasserstoffen durchgeführt werden. Als Phasentransferkatalysatoren können Ammoniumhalogenide und -tetrafluoroborate wie z. B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat sowie Phosphiniumhalogenide wie Tetrabutylphosphiniumchlorid oder Tetraphenylphosphoniumbromid eingesetzt werden.
C2) die Veretherung des Naphthols in zwei Stufen (Umsetzung von V über VII zu I). 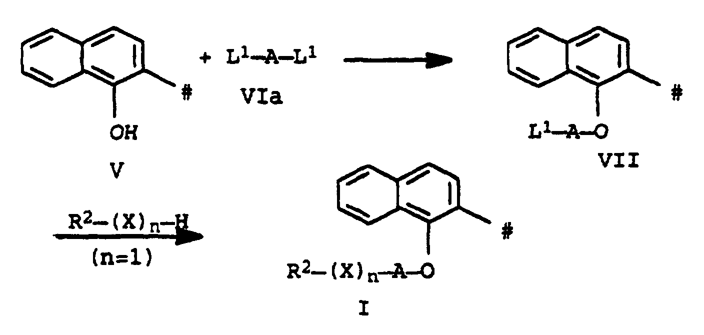
   Die entsprechenden Verbindungen I, in denen Y für ein ggf. subst. Stickstoffatom steht, erhält man durch Umsetzung von Verbindungen V, VII oder I, in denen Y für Sauerstoff steht, mit einem entsprechenden Amin in an sich bekannter Weise (Houben-Weyl, Vol. E5, S. 983ff).

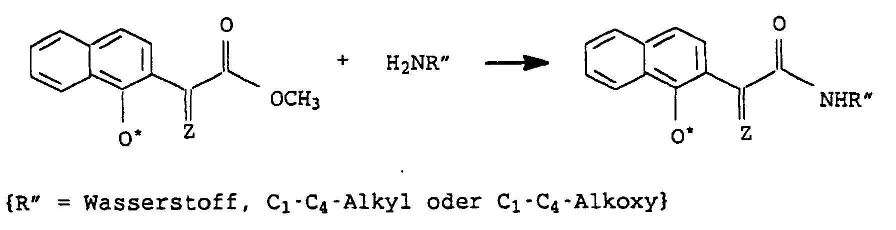

Diese Umsetzung wird üblicherweise bei Temperaturen von 0°C bis 60°C, vorzugsweise 10°C bis 40°C durchgeführt.

Methylamin kann durch gasförmiges Einleiten oder als wäßrige Lösung zu einer Lösung von II zudosiert werden.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol, besonders bevorzugt Methanol, Toluol und Tetrahydrofuran.

Es können auch Gemische der genannten Lösungsmittel verwendet werden.

Im Hinblick auf ihre biologische Wirkung gegen tierische Schädlinge und Schadpilze kommen Verbindungen der Formel I in Betracht, in denen der Index und die Substituenten die folgende Bedeutung haben:
- A: eine der Gruppen A.1 bis A.42 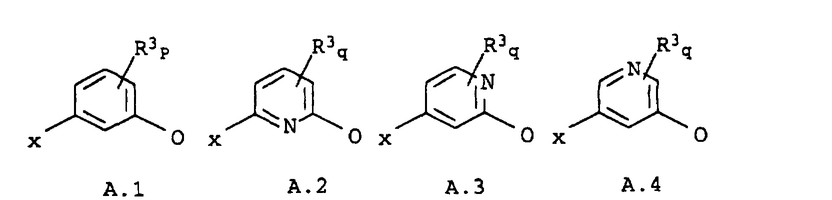 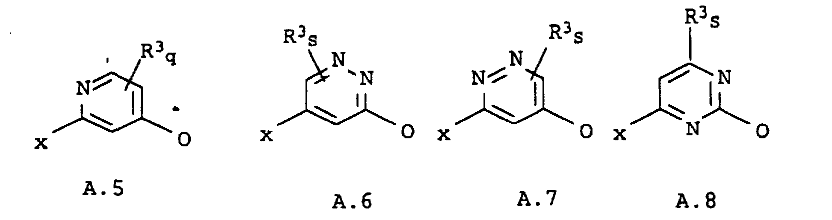 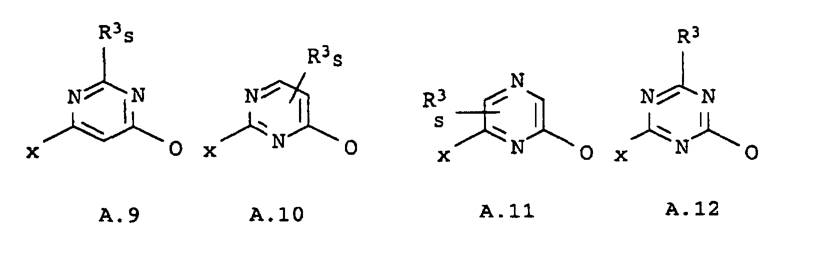 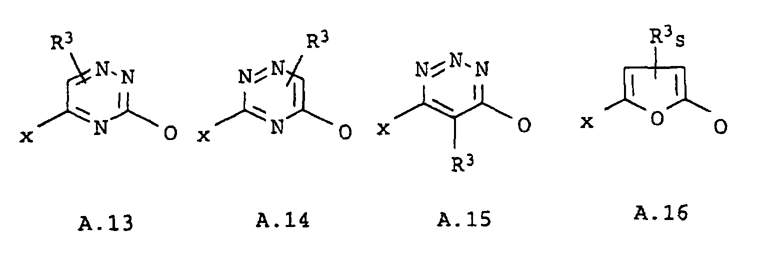 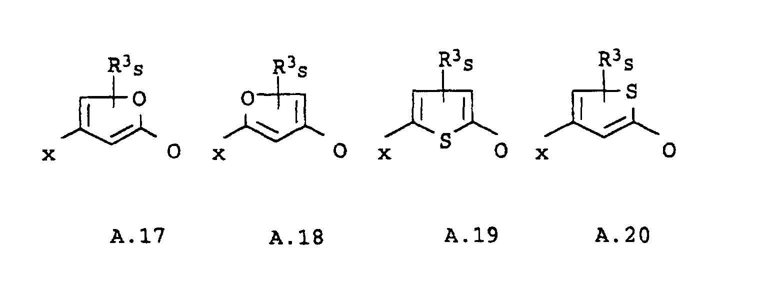 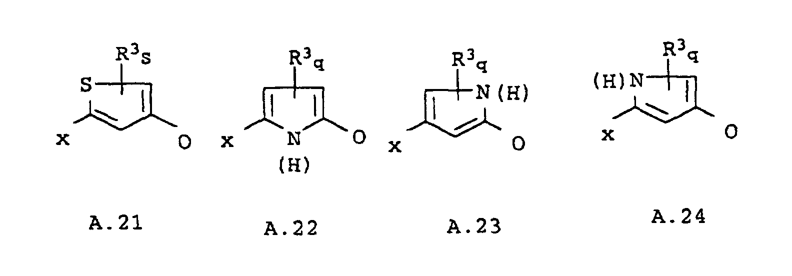 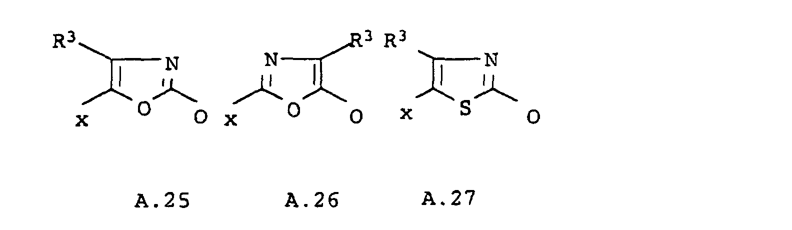 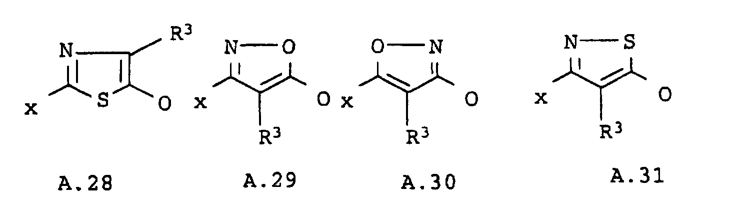 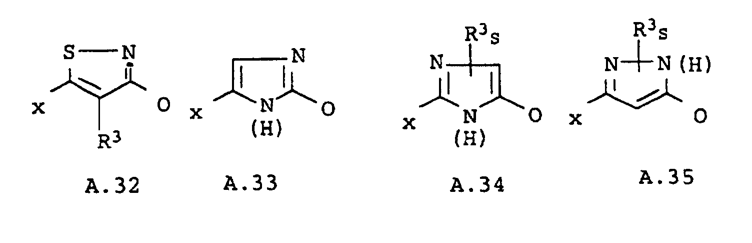 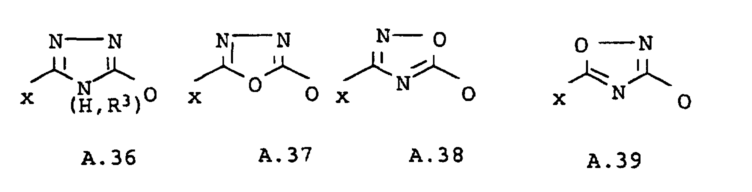 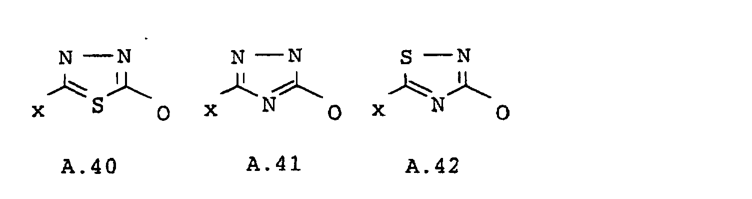
worin die Bezeichnungen x- und -o dazu dienen anzugeben, zu welchem der A benachbarten Reste die Bindung gerichtet ist, nämlich die mit x- Bezeichnete Bindung zum Rest -(X)ₙR² und die mit -o bezeichnete Bindung zum Sauerstoffatom, und worin
- p: 0, 1, 2, 3 oder 4 bedeutet, wobei die Reste R³ verschieden sein können, wenn p > 1 ist;
- q: 0, 1, 2 oder 3 bedeutet, wobei die Reste R³ verschieden sein können, wenn q > 1 ist;
- s: 0, 1 oder 2 bedeutet, wobei die Reste R³ verschieden sein können, wenn s > 1 ist;
wobei die Reste R³ an ein C-Atom des Rings oder, im Fall der Reste A.22, A.23, A.24, A.33, A.34, A.35 und A.36 zusätzlich auch an ein N-Atom des Rings gebunden sind und
- R³: die folgende Bedeutung hat:
Wasserstoff, Halogen, C₁-C₄-Alkyl, C₁-C₄-Halogenalkyl, C₁-C₄- Halogenalkoxy und C₃-C₆-Cycloalkyl;
- n: 0 oder 1;
- X: Sauerstoff, Schwefel oder Stickstoff, wobei das Stickstoffatom einen der folgenden Reste trägt: Wasserstoff, C₁-C₄-Alkyl, C₁-C₄-Alkoxy, C₃-C₆-Cycloalkyl, Phenyl oder Benzyl, wobei der Phenylring und der Benzylrest ein bis fünf Halogenatome oder ein bis drei der folgenden Reste tragen können: Halogen C₁-C₄-Alkyl, C₁-C₄-Halogenalkyl, C₁-C₄-Alkoxy, C₁-C₄- Halogenalkoxy, C₃-C₆-Cycloalkyl, Phenyl oder Benzyl;
- Y: Sauerstoff, eine direkte Bindung oder
für den Fall, daß Z für NOCH₃ steht: Sauerstoff, eine direkte Bindung oder Stickstoff, wobei das Stickstoffatom einen der folgenden Reste trägt: Wasserstoff, C₁-C₄-Alkyl oder C₁-C₄- Alkoxy;
- Z: CHOCH₃, NOCH₃, CHCH₃ oder CHCH₂CH₃;
- R¹: C₁-C₄-Alkyl
- R²: C₁-C₆-Alkyl, C₃-C₆-Cycloalkyl, C₂-C₆-Alkenyl oder C₃-C₆-Alkinyl, wobei diese Gruppen partiell oder vollständig halogeniert sein können und/oder einen oder zwei der folgenden Reste tragen können:
Hydroxy, Cyano, Isocyano, Nitro, Formyl, Amino, Formylamino, Carboxyl, Sulfoxyl, Carbonylamino, C₁-C₈ -Alkylamino, Di-C₁-C₈-alkylamino, C₁-C₈-Alkylcarbonyl, C₁-C₈-Alkoxycarbonyl, C₁-C₈-Alkylaminocarbonyl, C₁-C₈-Alkylsulfonyl oder Phenylsulfonyl;
Phenyl, welches ein bis fünf Halogenatome und/oder ein bis drei der folgenden Reste tragen kann:
Amino, Carboxyl, Carbonylamino, Cyano, Formyl, Formylamino, Hydroxy, Isocyano, Nitro, Sulfoxyl, Thiocarboxamido, Thiocyanato, C₁-C₈-Alkyl, C₁-C₄-Halogenalkyl, C₁-C₈-Alkylcarbonyl, C₁-C₈-Alkylcarbonyloxy, C₁-C₈-Alkylcarbonylthio, C₁-C₈-Alkylcarbonylamino, C₁-C₈-Alkylsulfonyl, C₁-C₈-Alkylsulfonyloxy, C₁-C₈-Alkylsulfonylamino, C₁-C₈-Alkoxy, C₁-C₄-Halogenalkoxy, C₁-C₈-Alkoxycarbonyl, C₁-C₆-Alkoxyiminoalkyl, C₁-C₈-Alkylthio, C₁-C₈-Alkylthiocarbonyl, C₁-C₈-Alkylamino, C₁-C₈-Alkylaminocarbonyl, Di-C₁-C₈-alkylamino, Di-C₁-C₈-alkylaminocarbonyl, C₃-C₈-Cycloalkyl, C₃-C₈-Cycloalkoxy, C₃-C₈-Cycloalkylthio, C₃-C₈-Cycloalkylamino, C₂-C₁₀-Alkenyl, C₂-C₈-Alkenyloxy, C₂-C₁₀-Alkinyl, C₂-C₁₀-Alkinyloxy, Phenylsulfonyl, Phenylsulfonyloxy oder Phenylsulfonylamino;
Furyl, Thienyl, Pyrrolyl, Pyrazolyl, Imidazolyl, Triazolyl, Isoxazolyl, Oxazolyl, Oxadiazolyl, Isothiazolyl, Thiazolyl, Thiadiazolyl, Pyridyl, Pyridazinyl, Pyrimidyl, Pyrazinyl oder Triazinyl, wobei diese Ringe ein bis vier Halogenatome und/oder ein bis drei der folgenden Reste tragen können:
Amino, Carboxyl, Carbonylamino, Cyano, Formyl, Formylamino, Hydroxy, Isocyano, Nitro, Sulfoxyl, Thiocarboxamido, Thiocyanato, C₁-C₈-Alkyl, C₁-C₄-Halogenalkyl, C₁-C₈-Alkylcarbonyl, C₁-C₈-Alkylcarbonyloxy, C₁-C₈-Alkylcarbonylthio, C₁-C₈-Alkylcarbonylamino, C₁-C₈-Alkylsulfonyl, C₁-C₈-Alkylsulfonyloxy, C₁-C₈-Alkylsulfonylamino, C₁-C₈-Alkoxy, C₁-C₄-Halogenalkoxy, C₁-C₈-Alkoxycarbonyl, C₁-C₆-Alkoxyiminoalkyl, C₁-C₈-Alkylthio, C₁-C₈-Alkylthiocarbonyl, C₁-C₈-Alkylamino, C₁-C₈-Alkylaminocarbonyl, Di-C₁-C₈-alkylamino, Di-C₁-C₈-alkylaminocarbonyl, C₃-C₈-Cycloalkyl, C₃-C₈-Cycloalkoxy, C₃-C₈-Cycloalkylthio, C₃-C₈-Cycloalkylamino, C₂-C₁₀-Alkenyl, C₂-C₈-Alkenyloxy, C₂-C₁₀-Alkinyl, C₂-C₁₀-Alkinyloxy, Phenylsulfonyl, Phenylsulfonyloxy oder Phenylsulfonylamino;
oder, für den Fall, daß der Wert von n 0 beträgt, bedeutet R² zusätzlich zu den vorstehenden Bedeutungen: Halogen, C₁-C₆-Alkoxy, C₁-C₄-Alkylsulfoxyl oder Phenylsulfoxyl, wobei der Phenylring seinerseits ein bis fünf Halogenatome und/oder ein bis drei der folgenden Substituenten tragen kann: Halogen, C₁-C₄-Alkyl, C₁-C₄-Halogenalkyl, C₁-C₄-Alkoxy, C₁-C₄-Halogenalkoxy, C₃-C₆-Cycloalkyl, Phenyl oder Benzyl.

Bei den vorstehenden Definitionen der Substituenten wurden Sammelbegriffe verwendet, die allgemein repräsentativ für die folgenden Gruppen stehen:
Halogen: Fluor, Chlor, Brom und Jod;
Alkyl: geradkettige oder verzweigte Alkylgruppen mit 1 bis 8, vorzugsweise 1 bis 6, insbesondere 1 bis 4 Kohlenstoffatomen, z.B. Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1- Ethyl-1-methylpropyl und 1-Ethyl-2-methylpropyl;
Halogenalkyl: geradkettige oder verzweigte Alkylgruppen mit 1 bis 4 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluor ethyl, 2,2-Dichlor-2-fluorethyl, 2,2,2-Trichlorethyl und Pentafluorethyl;
Alkylcarbonyl: geradkettige oder verzweigte Alkylgruppen mit 1 bis 8 Kohlenstoffatomen wie vorstehend im allgemeinen und im besonderen genannt, welche über eine Carbonylgruppe (-CO-) an das Gerüst gebunden sind;
Alkylcarbonyloxy: geradkettige oder verzweigte Alkylgruppen mit 1 bis 8 Kohlenstoffatomen wie vorstehend im allgemeinen und im besonderen genannt, welche über eine Carbonyloxygruppe (-CO-O-) an das Gerüst gebunden sind;
Alkylcarbonylthio: geradkettige oder verzweigte Alkylgruppen mit 1 bis 8 Kohlenstoffatomen wie vorstehend im allgemeinen und im besonderen genannt, welche über eine Carbonylthiogruppe (-CO-S-) an das Gerüst gebunden sind;
Alkylcarbonylamino: geradkettige oder verzweigte Alkylgruppen mit 1 bis 8 Kohlenstoffatomen wie vorstehend im allgemeinen und im besonderen genannt, welche über eine Carbonylaminogruppe (-CO-NH-) an das Gerüst gebunden sind;
Alkylsulfonyl: geradkettige oder verzweigte Alkylgruppen mit 1 bis 8 Kohlenstoffatomen wie vorstehend im allgemeinen und im besonderen genannt, welche über eine Sulfonylgruppe (-SO₂-) an das Gerüst gebunden sind;
Alkylsulfonyloxy: geradkettige oder verzweigte Alkylgruppen mit 1 bis 8 Kohlenstoffatomen wie vorstehend im allgemeinen und im besonderen genannt, welche über eine Sulfonyloxygruppe (-SO₃-) an das Gerüst gebunden sind;
Alkylsulfonylamino: geradkettige oder verzweigte Alkylgruppen mit 1 bis 8 Kohlenstoffatomen wie vorstehend im allgemeinen und im besonderen genannt, welche über eine Sulfonylaminogruppe ( -SO₂NH-) an das Gerüst gebunden sind;
Alkoxy: geradkettige oder verzweigte Alkoxygruppen mit 1 bis 8, vorzugsweise 1 bis 6, insbesondere 1 bis 4 Kohlenstoffatomen, z.B. Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methyl pentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
Halogenalkoxy: geradkettige oder verzweigte Alkoxygruppen mit 1 bis 4 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
Alkoxycarbonyl: geradkettige oder verzweigte Alkoxygruppen mit 1 bis 8 Kohlenstoffatomen wie vorstehend im allgemeinen und im besonderen genannt, welche über eine Carbonylgruppe (-CO-) an das Gerüst gebunden sind;
Alkoxyiminoalkyl: geradkettige oder verzweigte Alkylgruppen mit 1 bis 6 Kohlenstoffatomen wie vorstehend genannt, welche in 1-Position einer Alkylgruppe, vorzugsweise einer C₁-C₆-Alkylgruppe, insbesondere einer C₁-C₄-Alkylgruppe, eine Alkoxyiminogruppe (Alkoxy-N=), vorzugsweise C₁-C₆-Alkoxy-N=, tragen;
Alkylthio: geradkettige oder verzweigte Alkylthiogruppen mit 1 bis 8, vorzugsweise 1 bis 6, insbesondere 1 bis 4, Kohlenstoffatomen, z.B. Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methyl pentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
Alkylthiocarbonyl: geradkettige oder verzweigte Alkylgruppen mit 1 bis 8 Kohlenstoffatomen wie vorstehend im allgemeinen und im besonderen genannt, welche über eine Thiocarbonylgruppe (-S-CO-) an das Gerüst gebunden sind;
Alkylamino: eine Aminogruppe, welche eine geradkettige oder verzweigte Alkylgruppe mit 1 bis 8 Kohlenstoffatomen wie vorstehend im allgemeinen und im besonderen genannt, trägt;
Alkylaminocarbonyl: geradkettige oder verzweigte Alkylgruppen mit 1 bis 8 Kohlenstoffatomen wie vorstehend im allgemeinen und im besonderen genannt, welche über eine Aminocarbonylgruppe (-NH-CO-) an das Gerüst gebunden sind;
Dialkylamino: eine Aminogruppe, welche zwei voneinander unabhängige, geradkettige oder verzweigte Alkylgruppen mit jeweils 1 bis 8, vorzugsweise 1 bis 6, insbesondere 1 bis 4 Kohlenstoffatomen trägt, z.B. Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-meth ylpropyl)amino, N,N-Di-(1,1-dimethylethyl)amino, N-Ethyl-N-meth ylamino, N-Methyl-N-propylamino, N-Methyl-N-(1-methylethyl)amino, N-Butyl-N-methylamino, N-Methyl-N-(1-methylpropyl)amino, N-Meth yl-N-(2-methylpropyl)amino, N-(1,1-Dimethylethyl)-N-methylamino, N-Ethyl-N-propylamino, N-Ethyl-N-(1-methylethyl)amino, N-ButylN-ethylamino, N-Ethyl-N-(1-methyl-propyl)amino, N-EthylN-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Di-methylethyl)-N-(1-methylethyl) amino, N-Butyl-N-(1-methyl-propyl)amino, N-Butyl-N-(2-methylpropyl)amino, N-Butyl-N-(1,1-dimethylethyl)amino, N-(1-Methylpropyl)-N-(2-methyl-propyl)amino, N- (1,1-Dimethylethyl)-N-(1-methylpropyl)amino und N-(1,1-Dimethylethyl)-N- (2-methylpropyl) amino;
Dialkylaminocarbonyl: eine Aminogruppe, welche zwei voneinander unabhängige, geradkettige oder verzweigte Alkylgruppen wie vorstehend im allgemeinen und im besonderen genannt mit jeweils 1 bis 8 Kohlenstoffatomen trägt und über eine Carbonylgruppe (-CO-) an das Gerüst gebunden ist;
Cycloalkyl: monocyclische Alkylgruppen mit 3 bis 8, vorzugsweise 3 bis 6 Kohlenstoffringgliedern, wie Cyclopropyl, Cyclobutyl, Cyclopentyl, Cyclohexyl, Cycloheptyl und Cyclooctyl;
Cycloalkoxy: monocyclische Alkylgruppen mit 3 bis 8, vorzugsweise 3 bis 6 Kohlenstoffringgliedern, welche über eine Oxygruppe (-O-) an das Gerüst gebunden sind;
Cycloalkamino: monocyclische Alkylgruppen mit 3 bis 8 Kohlenstoffringgliedern, welche über eine Aminogruppe (-NH-) an das Gerüst gebunden sind;
Alkenyl: geradkettige oder verzweigte Alkenylgruppen mit 2 bis 10, vorzugsweise 2 bis 8, insbesondere 2 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl. 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2- butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3- butenyl, 3,3-Dimethyl-1-butenyl, 3,3-Dimethyl-2-butenyl, 1-Eth yl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-prop enyl und 1-Ethyl-2-methyl-2-propenyl;
Alkenyloxy: geradkettige oder verzweigte Alkenyloxygruppen mit 2 bis 8 Kohlenstoffatomen und einer Doppelbindung in einer beliebigen Position, z.B. C₂-C₆-Alkenyloxy wie Ethenyloxy, 1-Propenyloxy, 2-Propenyloxy, 1-Methylethenyloxy, 1-Butenyloxy, 2-Butenyloxy, 3-Butenyloxy, 1-Methyl-l-propenyloxy, 2-Meth yl-1-propenyloxy, 1-Methyl-2-propenyloxy, 2-Methyl-2-propenyloxy , 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Di-methyl-3-butenyloxy, 1,2-Dimethyl-1-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy und 1-Ethyl-2-methyl-2-propenyloxy;
Alkinyl: geradkettige oder verzweigte Alkinylgruppen mit 2 bis 10, vorzugsweise 2 bis 8, insbesondere 2 bis 6 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Di-methyl-3-bu tinyl, 1,2-Dimethyl-3-butinyl, 2,2-Dimethyl-3-butinyl, 3,3-Dime thyl-1-butinyl, 1-Ethyl-2-butinyl, 1-Ethyl-3-butinyl, 2-Ethyl-3 -butinyl und 1-Ethyl-1-methyl-2-propinyl;
Alkinyloxy: geradkettige oder verzweigte Alkinyloxygruppen mit 2 bis 10, vorzugsweise 2 bis 8, insbesondere 2 bis 6 Kohlenstoffatomen und einer Doppelbindung in einer beliebigen Position, z.B. C₂-C₆-Alkinyloxy wie Ethinyloxy, 1-Propinyloxy, 2-Propinyloxy, 1-Butinyloxy, 2-Butinyloxy, 3-Butinyloxy, 1-Methyl-2-propin yloxy, 1-Pentinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 3-Methyl-1-butinyloxy, 1,1-Dimethyl-2-propinyl oxy, 1-Ethyl-2-propinyloxy, 1-Hexinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-l-pentinyloxy, 3-Methyl-4-pentinyloxy, 4-Methyl-1-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 3,3-Dimethyl-1-butinyloxy, l-Ethyl-2-butinyloxy, 1-Ethyl-3-butinyloxy, 2-Ethyl-3-butinyloxy und 1-Ethyl-1-methyl-2-propinyloxy;
Phenylsulfonyl, Phenylsulfonyloxy und Phenylsulfonylamino: Phenylreste, die über eine Sulfonylgruppe (-SO₂-), eine Sulfonyloxygruppe (-SO₃-) bzw. eine Sulfonylaminogruppe (-SO₂-NH-) an das Gerüst gebunden sind.

Besonders bevorzugt sind Verbindungen I, in denen A für einen der Reste A.1, A.2, A.3, A.4, A.5, A.8, A.9, A.10, A.11, A.12 oder A.15 steht.

Außerdem werden Verbindungen I bevorzugt, in denen A unsubstituiert ist oder ein oder zwei der folgenden Reste R³ trägt: Cyano, Nitro, Thiocarboxamide, Halogen, vorzugsweise Fluor oder Chlor, insbesondere Chlor; C₁-C₄-Alkyl, vorzugsweise C₁-C₃-Alkyl, insbesondere Methyl; C₁-C₂-Halogenalkyl, insbesondere Trifluormethyl. Daneben werden Verbindungen I bevorzugt, in denen Z CHOCH₃, NOCH₃ oder CHCH₃ bedeutet.

Desweiteren sind Verbindungen I bevorzugt, in denen Y Sauerstoff bedeutet.

Außerdem werden Verbindungen I bevorzugt, in denen Z NOCH₃ bedeutet und Y für Stickstoff steht, wobei das Stickstoffatom einen der folgenden Reste trägt:
- Wasserstoff;
- C₁-C₄-Alkyl, vorzugsweise C₁-C₂-Alkyl, insbesondere Methyl oder
- C₁-C₄-Alkoxy, vorzugsweise C₁-C₂-Alkoxy, insbesondere Methoxy.

Die Naphthylether der Formel I können bedingt durch die Herstellung aufgrund der C=C- bzw. C=N-Doppelbindungen als E/Z-Isomerengemische anfallen. Die isomeren Verbindungen können in üblicher Weise durch Kristallisation oder Chromatographie in die isomerenreinen Verbindungen aufgetrennt werden. Sowohl die Isomerengemische als auch die isomerenreinen Verbindungen können in der erfindungsgemäßen Weise zur Bekämpfung von tierischen Schädlingen und Schadpilzen verwendet werden.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Tragerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsaure-di-isopropylester; heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)phosphinyl]-3-phenyl-1,2,4- triazol, 2,3-Dicyano-1,4-dithioanthrachinon, 2-Thio-1,3- dithiolo[4,5-b]chinoxalin, 1-(Butylcarbamoyl)-2- benz- imidazol-carbaminsäuremethylester, 2-Methoxycarbonylamino- benzimidazol, 2-(Furyl-(2))-benzimidazol, 2-(Thiazolyl-(4))-benzimidazol, N-(1,1,2,2-Tetra-chlorethylthio)-tetrahydrophthalimid, N-Trichlormethylthiotetrahydrophthalimid, N-Trichlormethylthio-phthalimid, N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan- 3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis- (1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4- n-propyl-1,3-dioxolan-2-yl-ethyl]-1H- 1,2,4-triazol, N-(n-Propyl)-N-(2,4,6-trichlorphenoxyethyl)-N'-imidazol-yl- harnstoff, 1-(4-Chlorphenoxy)-3,3-dimethyl-1-(1H-1,2,4-triazol-1-yl)-2-butanon, 1-(4-Chlorphenoxy)-3,3- dimethyl-1-(1H-1,2,4-triazol-1-yl)-2- butanol, α-(2-Chlorphenyl)-α-(4-chlorphenyl)-5 -pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, sowie verschiedene Fungizide, wie Dodecylguanidinacetat, 3-[3- (3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl) -N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropyl-carbamoylhydantoin, N-(3,5-Dichlorphenyl)-1,2-dimethylcyclopropan-1,2-dicarbonsäure- imid, 2-Cyano-[N-(ethylaminocarbonyl)-2-methoximino]-acetamid, 1-[2-(2,4-Dichlorphenyl)-pentyll-lH-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)methyl)-1H-1,2,4-triazol.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen in Abhängigkeit vom Bekämpfungsziel 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Staubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile der Verbindung Nr. 1.001 werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile der Verbindung Nr. 1.003 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile der Verbindung Nr. 1.001 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile der Verbindung Nr. 1.002 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile der Verbindung Nr. 1.002 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile der Verbindung Nr. 1.001 mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile der Verbindung Nr. 1.002 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII. 20 Gew.-Teile des Wirkstoffs Nr. 1.003 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

### Herstellungsbeispiele

1. Herstellung von 1-Hydroxy-2-naphthylglyoxylsäuremethylester 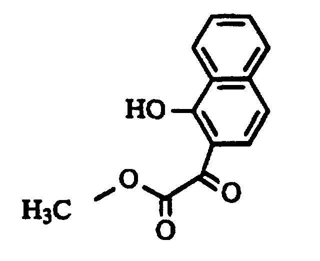 Bei -10°C werden unter Stickstoff 28,5 g (0,15 mol) Titantetrachlorid zu 150 ml Methylenchlorid gegeben. Dann werden bei-40°C zuerst 22,3 g (0,15 mol) 1-Naphthol, gelöst in 100 ml Methylenchlorid und anschließend 18,5 g (0,15 mol) Oxalsäuremethylesterchlorid zugetropft. Man rührt noch 30 min bei dieser Temperatur, gibt dann bei Raumtemperatur (20°C) etwas verdünnte Salzsäure zu und gießt auf Eis. Die organische Phase wird 1 x mit H₂O gewaschen, über Natriumsulfat getrocknet und eingeengt. Man erhält 30 g (0,13 mol) 1-Hydroxy-2-naphtylglyoxylsäuremethylester (87 % Ausbeute).
   ¹H-NMR (CDCl₃): δ = 4.05(s,3H), 7.25(d,1H), 7.40-7.80(m,4H), 8.45(d,1H), 13.00 ppm (s,1H).
2. Herstellung von 2-Hydroxy-2-naphthylglyoxylsauremethylester-O-methyloxim 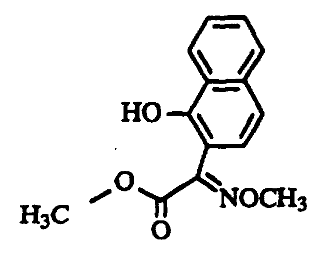 69 g (0,26 mol) l-Hydroxy-2-naphthylglyoxylsäuremethylester und 44 g (0,53 mol) O-Methylhydroxylaminhydrochlorid werden in 800 ml Methanol vorgelegt und 6 h unter Rühren auf Rückflußtemperatur erhitzt. Dann wird eingeengt, der Rückstand in Essigester aufgenommen, mit H₂O gewaschen, über Natriumsulfat getrocknet und erneut eingeengt. Man erhält 52 g (0,20 mol) 1-Hydroxy-2-naphthylglyoxylsäuremethylester-O-methyloxim (77 % Ausbeute).
   ¹H-NMR (CDCl₃): δ = 4.00(s,3H), 4.05(s,3H), 7.00(d,1H), 7.30 (d,1H), 7.40-7.90(m,4H), 8.35(m,1H), 10.90 ppm (s,1H).
3. Herstellung von 1-[6-Chlor-pyrimidin-4-yl-oxy]-naphthyl-2-glyoxylsauremethylester-O-methyloxim 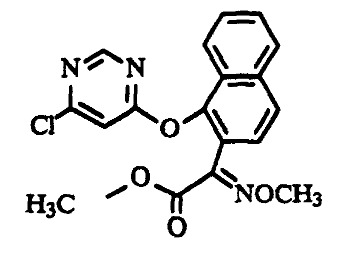 6 g (0,04 mol) 4,6-Dichlorpyrimidin und 7 g (0,05 mol) Kaliumcarbonat werden in 60 ml Dimethylformamid vorgelegt. Bei 0°C tropft man 7,7 g (0,03 mol) der Verbindung Beispiel 2 dazu, rührt 1 Stunde bei 0°C und dann über Nacht bei RT weiter. Dann wird auf Eis gegossen und mit Ether extrahiert. Die organische Phase wird mit Wasser gewaschen, über Natriumsulfat getrocknet und eingeengt. Man erhält 8 g (72 %) Produkt.
   ¹H-NMR (CDCl₃): δ = 3.81(s,3H), 4.0(s,3H), 6.90(s,1H), 7.4-8.0(m,6H) und 8.50 ppm (s,1H).
4. Herstellung von 1-[6-(2-Cyanophenoxy)pyrimidin-4-yl-oxy]-naphthyl-2-glyoxylsäure-methylester-O-methyloxim
   3,5 g (9 mmol) der Verbindung Beispiel 3 werden zusammen mit 1,2 g (10 mmol) 2-Cyanophenol, 1,4 g (10 mmol) Kaliumcarbonat und 100 mg Kupfer(I)-chlorid in 100 ml DMF 5 Stunden bei 100°C gerührt. Dann wird auf Eis gegossen und mit Ether extrahiert. Die organische Phase wird mit verdünnter Natronlauge gewaschen, über Natriumsulfat getrocknet und eingeengt.
   Es bleiben 3 g (73 %) Produkt zurück. 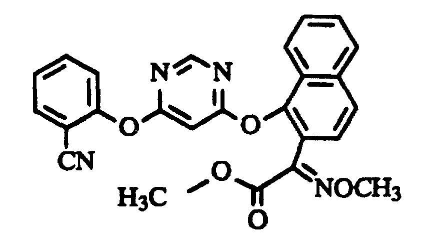 ¹H-NMR (CDCl₃): δ = 3.95(s,3H), 4.0(s,3H), 6.57(s,1H), 7.22-8.0(m,10H) und 8.3 ppm (s,1H).

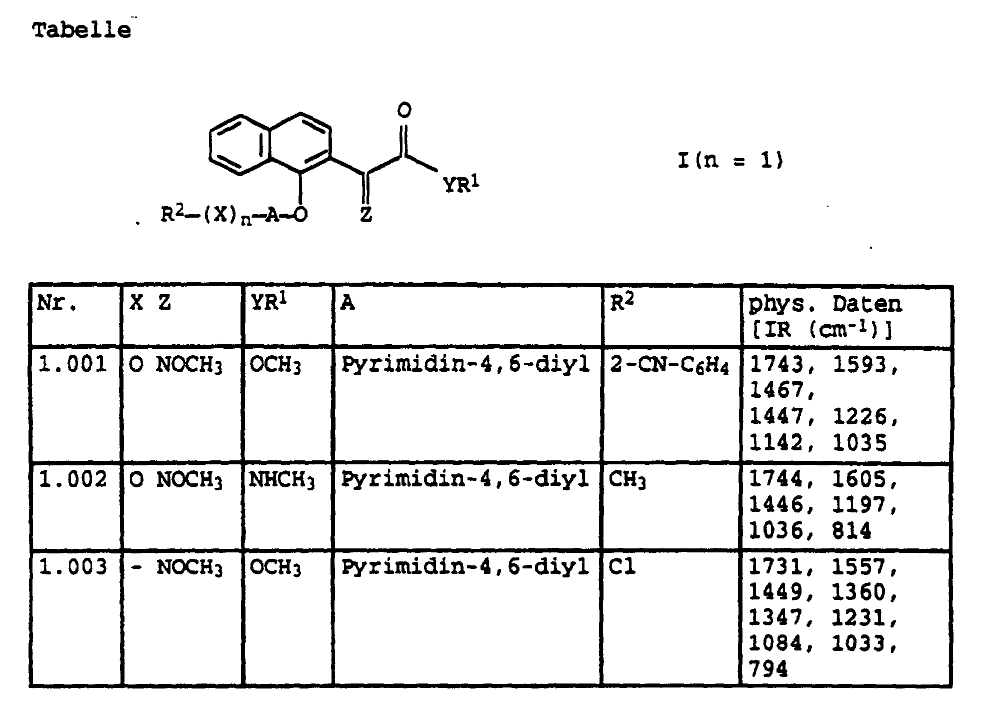

### Beispiele zur biologischen Wirkung

### Fungizide Wirksamkeit

### Botrytis cinera

Paprikasämlinge der Sorte "Neusiedler Ideal Elite" mit 4-5 gut entwickelten Blättern wurden mit einer wäßrigen Wirkstoffsuspension troßfnaß gespritzt. Nach dem Antrocknen des Spritzbelags wurden die Pflanzen mit einer Konidienaufschwemmung des Pilzes Botrytis cinera besprüht. Nach 5 Tagen bei 22 bis 24°C und hoher Luftfeuchtigkeit wurde der Pilz-Befall der Blätter beurteilt.

In diesem Text zeigten die mit 250 ppm der Verbindungen 1.001 und 1.003 behandelten Pflanzen einen Befall von 5 %, während bei den unbehandelten Pflanzen 70 % der Blattflächen befallen waren.

### Erysiphe graminis var. tritici (Weizenmehltau)

Weizenkeimlinge der Sorte "Frühgold" wurden mit einer wäßrigen Wirkstoffsuspension troßfnaß gespritzt. Nach 24 h wurden die Pflanzen mit Oiden (Sporen) des Pilzes Erxsiphe graminis var. tritici (Weizenmehltau) bestäubt. Nach 7 Tagen bei 22 bis 24°C und 75 bis 80 % Luftfeuchtigkeit wurde der Pilz-Befall der Blätter beurteilt.

In diesem Test zeigten die mit 250 ppm der Verbindungen 1.001, 1.002 und 1.003 behandelten Pflanzen einen Befall von 15 % und weniger, während bei den unbehandelten Pflanzen 65 % der Blattflächen befallen waren.

## Patentansprüche

1. Naphthylether der allgemeinen Formel I 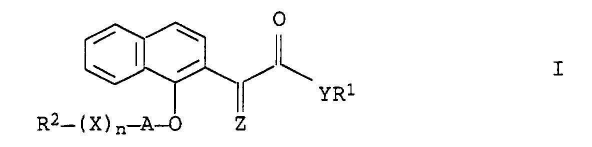 in der der Index und die Substituenten die folgende Bedeutung haben:
A eine der Gruppen A.1 bis A.42 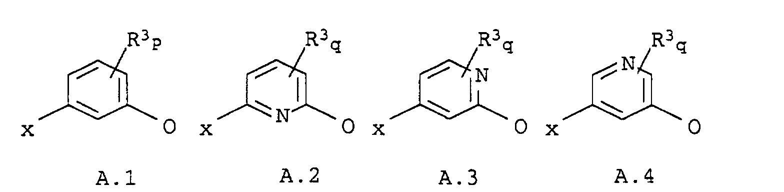 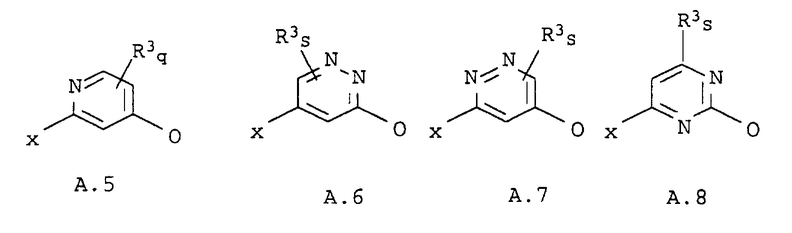 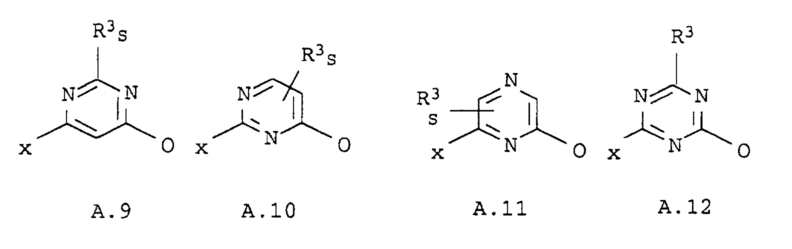 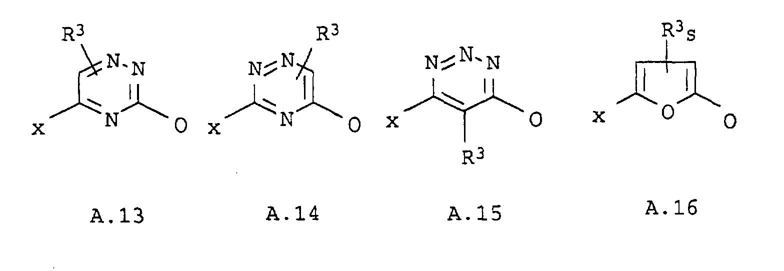 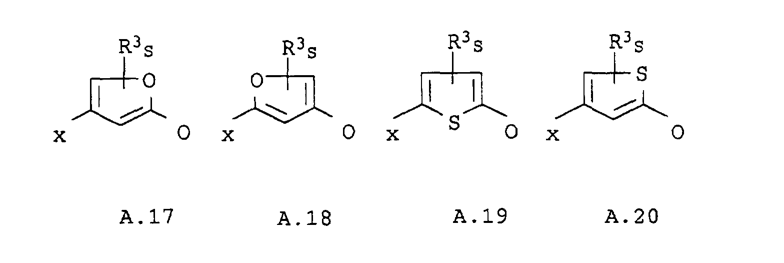 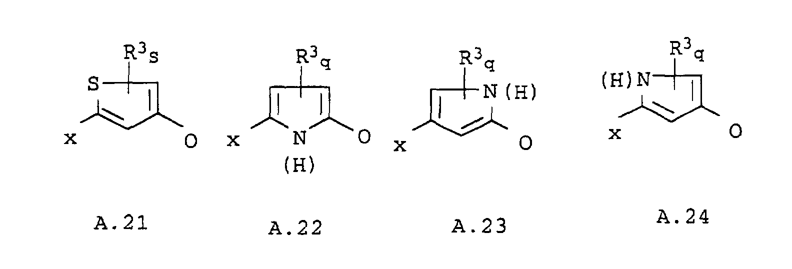 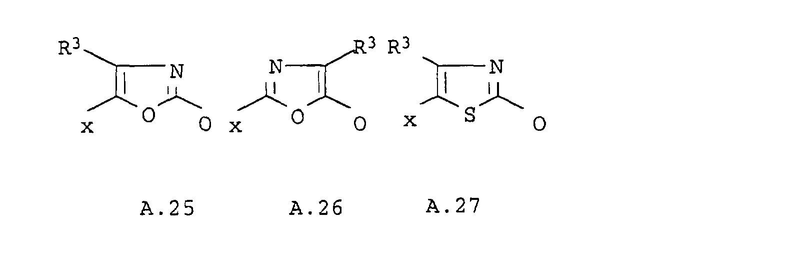 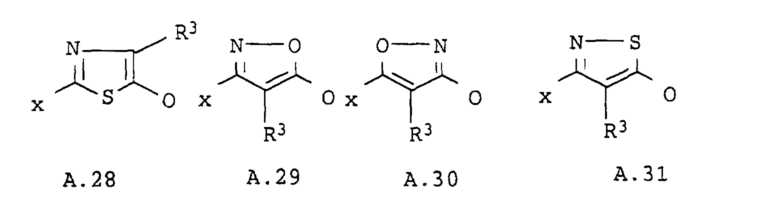 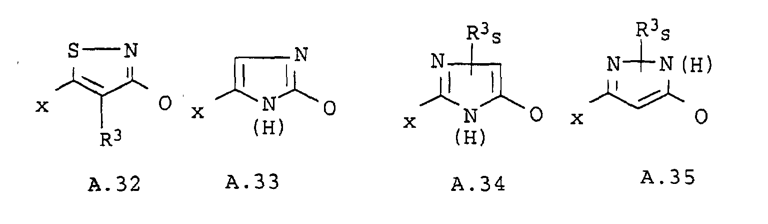 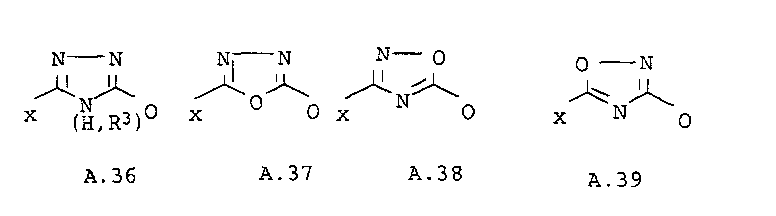 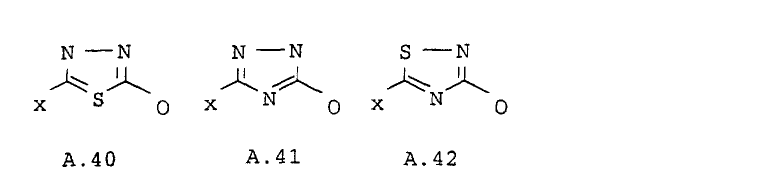
worin die Bezeichnungen x- und -o dazu dienen anzugeben, zu welchem der A benachbarten Reste die Bindung gerichtet ist, nämlich die mit x- Bezeichnete Bindung zum Rest -(X)ₙR² und die mit -o bezeichnete Bindung zum Sauerstoffatom, und worin
p 0, 1, 2, 3 oder 4 bedeutet, wobei die Reste R³ verschieden sein können, wenn p > 1 ist;
q 0, 1, 2 oder 3 bedeutet, wobei die Reste R³ verschieden sein können, wenn q > 1 ist;
s 0, 1 oder 2 bedeutet, wobei die Reste R³ verschieden sein können, wenn s > 1 ist;
wobei die Reste R³ an ein C-Atom des Rings oder, im Fall der Reste A.22, A.23, A.24, A.33, A.34, A.35 und A.36 zusätzlich auch an ein N-Atom des Rings gebunden sind und
R³ die folgende Bedeutung hat:
Wasserstoff, Halogen, C₁-C₄-Alkyl, C₁-C₄-Halogenalkyl, C₁-C₄- Halogenalkoxy und C₃-C₆-Cycloalkyl;
n 0 oder 1;
X Sauerstoff, Schwefel oder Stickstoff, wobei das Stickstoffatom einen der folgenden Reste trägt: Wasserstoff, C₁-C₄-Alkyl, C₁-C₄-Alkoxy, C₃-C₆-Cycloalkyl, Phenyl oder Benzyl, wobei der Phenylring und der Benzylrest ein bis fünf Halogenatome oder ein bis drei der folgenden Reste tragen können: Halogen C₁-C₄-Alkyl, C₁-C₄-Halogenalkyl, C₁-C₄-Alkoxy, C₁-C₄- Halogenalkoxy, C₃-C₆-Cycloalkyl, Phenyl oder Benzyl;
Y Sauerstoff, eine direkte Bindung oder
für den Fall, daß Z für NOCH₃ steht: Sauerstoff, eine direkte Bindung oder Stickstoff, wobei das Stickstoffatom einen der folgenden Reste trägt: Wasserstoff, C₁-C₄-Alkyl oder C₁-C₄- Alkoxy;
Z CHOCH₃, NOCH₃, CHCH₃ oder CHCH₂CH₃;
R¹ C₁-C₄-Alkyl
R² C₁-C₆-Alkyl, C₃-C₆-Cycloalkyl, C₂-C₆-Alkenyl oder C₃-C₆-Alkinyl, wobei diese Gruppen partiell oder vollständig halogeniert sein können und/oder einen oder zwei der folgenden Reste tragen können:
Hydroxy, Cyano, Isocyano, Nitro, Formyl, Amino, Formylamino, Carboxyl, Sulfoxyl, Carbonylamino, C₁-C₈-Alkylamino, Di-C₁-C₈-alkylamino, C₁-C₈-Alkylcarbonyl, C₁-C₈-Alkoxycarbonyl, C₁-C₈-Alkylaminocarbonyl, C₁-C₈-Alkylsulfonyl oder Phenylsulfonyl;
Phenyl, welches ein bis fünf Halogenatome und/oder ein bis drei der folgenden Reste tragen kann:
Amino, Carboxyl, Carbonylamino, Cyano, Formyl, Formylamino, Hydroxy, Isocyano, Nitro, Sulfoxyl, Thiocarboxamido, Thiocyanato, C₁-C₈-Alkyl, C₁-C₄-Halogenalkyl, C₁-C₈-Alkylcarbonyl, C₁-C₈-Alkylcarbonyloxy, C₁-C₈-Alkylcarbonylthio, C₁-C₈-Alkylcarbonylamino, C₁-C₈-Alkylsulfonyl, C₁-C₈-Alkylsulfonyloxy, C₁-C₈-Alkylsulfonylamino, C₁-C₈-Alkoxy, C₁-C₄-Halogenalkoxy, C₁-C₈-Alkoxycarbonyl, C₁-C₆-Alkoxyiminoalkyl, C₁-C₈-Alkylthio, C₁-C₈-Alkylthiocarbonyl, C₁-C₈-Alkylamino, C₁-C₈-Alkylaminocarbonyl, Di-C₁-C₈-alkylamino, Di-C₁-C₈-alkylaminocarbonyl, C₃-C₈-Cycloalkyl, C₃-C₈-Cycloalkoxy, C₃-C₈-Cycloalkylthio, C₃-C₈-Cycloalkylamino, C₂-C₁₀-Alkenyl, C₂-C₈-Alkenyloxy, C₂-C₁₀-Alkinyl, C₂-C₁₀-Alkinyloxy, Phenylsulfonyl, Phenylsulfonyloxy oder Phenylsulfonylamino;
Furyl, Thienyl, Pyrrolyl, Pyrazolyl, Imidazolyl, Triazolyl, Isoxazolyl, Oxazolyl, Oxadiazolyl, Isothiazolyl, Thiazolyl, Thiadiazolyl, Pyridyl, Pyridazinyl, Pyrimidyl, Pyrazinyl oder Triazinyl, wobei diese Ringe ein bis vier Halogenatome und/oder ein bis drei der folgenden Reste tragen können:
Amino, Carboxyl, Carbonylamino, Cyano, Formyl, Formylamino, Hydroxy, Isocyano, Nitro, Sulfoxyl, Thiocarboxamido, Thiocyanato, C₁-C₈-Alkyl, C₁-C₄-Halogenalkyl, C₁-C₈-Alkylcarbonyl, C₁-C₈-Alkylcarbonyloxy, C₁-C₈-Alkylcarbonylthio, C₁-C₈-Alkylcarbonylamino, C₁-C₈-Alkylsulfonyl, C₁-C₈-Alkylsulfonyloxy, C₁-C₈-Alkylsulfonylamino, C₁-C₈-Alkoxy, C₁-C₄-Halogenalkoxy, C₁-C₈-Alkoxycarbonyl, C₁-C₆-Alkoxyiminoalkyl, C₁-C₈-Alkylthio, C₁-C₈-Alkylthiocarbonyl, C₁-C₈-Alkylamino, C₁-C₈-Alkylaminocarbonyl, Di-C₁-C₈-alkylamino, Di-C₁-C₈-alkylaminocarbonyl, C₃-C₈-Cycloalkyl, C₃-C₈-Cycloalkoxy, C₃-C₈-Cycloalkylthio, C₃-C₈-Cycloalkylamino, C₂-C₁₀-Alkenyl, C₂-C₈-Alkenyloxy, C₂-C₁₀-Alkinyl, C₂-C₁₀-Alkinyloxy, Phenylsulfonyl, Phenylsulfonyloxy oder Phenylsulfonylamino;
oder, für den Fall, daß der Wert von n 0 beträgt, bedeutet R² zusätzlich zu den vorstehenden Bedeutungen: Halogen, C₁-C₆-Alkoxy, C₁-C₄-Alkylsulfoxyl oder Phenylsulfoxyl, wobei der Phenylring seinerseits ein bis fünf Halogenatome und/oder ein bis drei der folgenden Substituenten tragen kann: Halogen, C₁-C₄-Alkyl, C₁-C₄-Halogenalkyl, C₁-C₄-Alkoxy, C₁-C₄-Halogenalkoxy, C₃-C₆-Cycloalkyl, Phenyl oder Benzyl.

2. Verbindungen der Formel I gemäß Anspruch 1, in denen Y für Sauerstoff und Z für CHOCH₃, NOCH₃ oder CHCH₃ steht.

3. Verbindungen der Formel I gemäß Anspruch 1, in denen Y für NH steht und Z NOCH₃ bedeutet.

4. Verbindungen der Formel I gemäß Anspruch 2 oder Anspruch 3, in denen n den Wert 1 hat und R² für A1 steht, wobei A1 die in Anspruch 1 gegebene Bedeutung hat.

5. Mittel gegen tierische Schädlinge oder Schadpilze, enthaltend übliche Zusatzstoffe und eine wirksame Menge einer Verbindung der Formel I gemäß Anspruch 1.

6. Mittel nach Anspruch 5 zur Bekämpfung tierischer Schädlinge aus der Klasse der Insekten, Spinntiere oder Nematoden.

7. Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

8. Verwendung der Verbindungen I gemäß Anspruch 1 zur Herstellung von Mitteln gegen tierische Schädlinge oder Schadpilze.

9. Verwendung der Verbindungen I gemäß Anspruch 1 zur Bekämpfung von tierischen Schädlingen oder Schadpilzen.

## Claims

1. A naphthyl ether of the general formula I 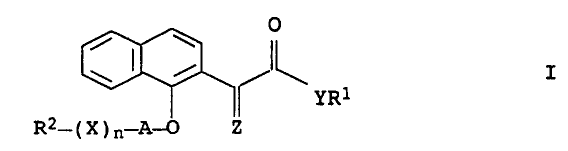 where the index and the substituents have the following meanings:
A is one of the groups A.1 to A.42 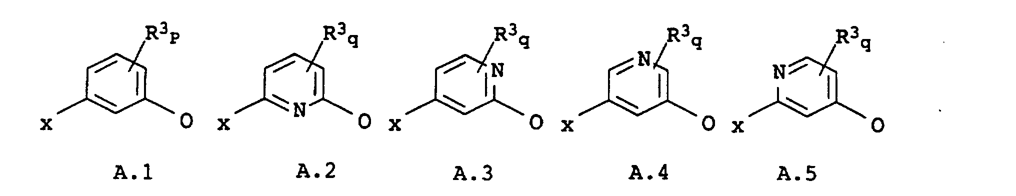 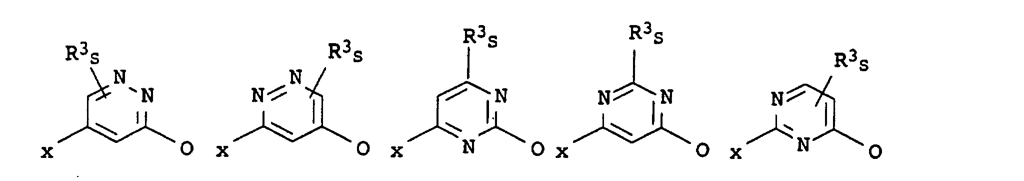 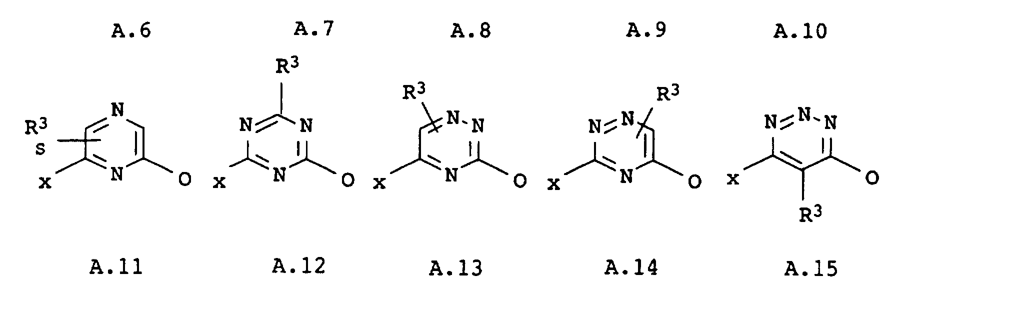 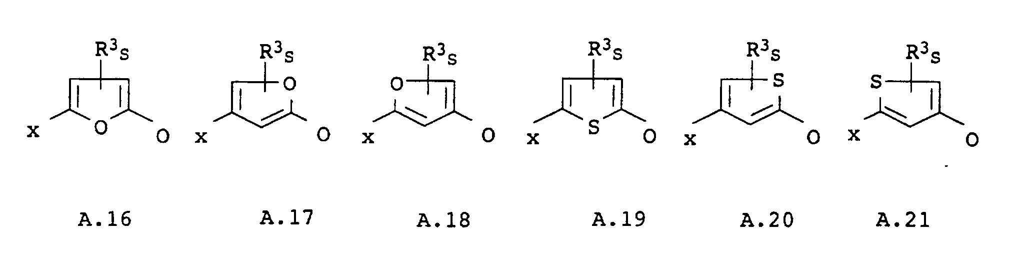 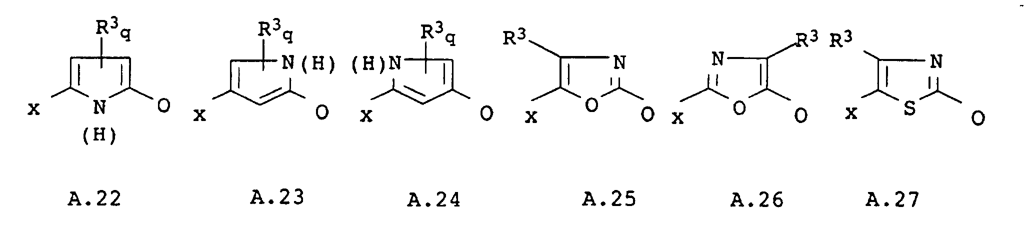 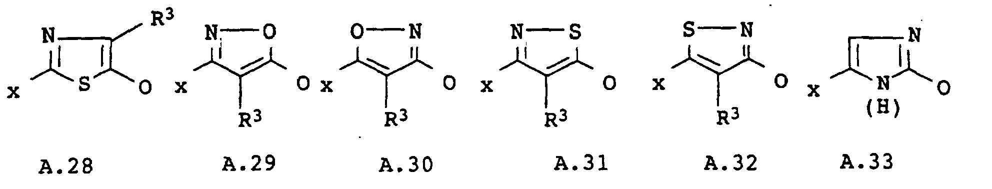 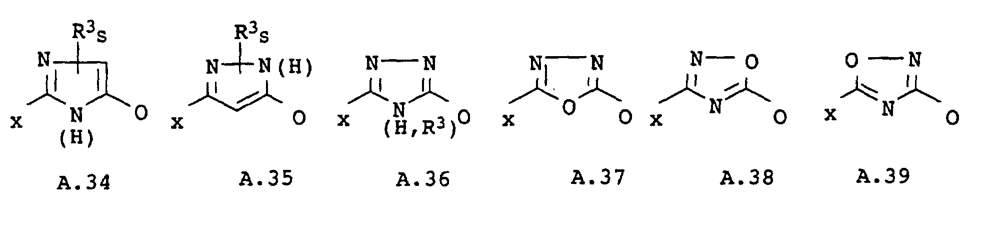 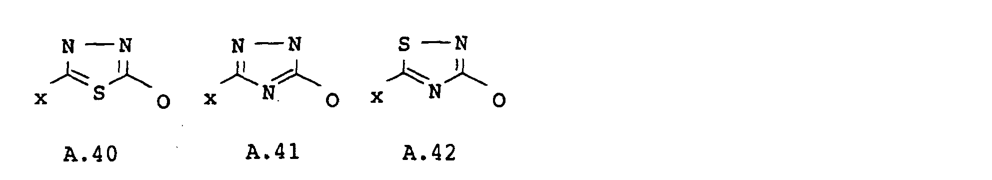
where the symbols x- and -o serve to indicate to which of the radicals adjacent to A the bond is directed, namely the bond designated by x- to the radical -(X)ₙR² and the bond designated by -o to the oxygen atom, and where
P is 0, 1, 2, 3 or 4, it being possible for the radicals R³ to be different if p > 1;
q is 0, 1, 2 or 3, it being possible for the radicals R³ to be different if q is > 1;
S is 0, 1 or 2, it being possible for the radicals R³ to be different if s is > 1;
the radicals R³ being bonded to a C atom of the ring or, in the case of the radicals A.22, A.23, A.24, A.33, A.34, A.35 and A.36, additionally to an N atom of the ring and
R³ has the following meanings:
hydrogen, halogen, C₁-C₄-alkyl, C₁-C₄-haloalkyl, C₁-C₄-haloalkoxy and C₃-C₆-cycloalkyl;
n is 0 or 1;
X is oxygen, sulfur or nitrogen, the nitrogen atom carrying one of the following radicals: hydrogen, C₁-C₄-alkyl, C₁-C₄-alkoxy, C₃-C₆-cycloalkyl, phenyl or benzyl, the phenyl ring and the benzyl radical being able to carry one to five halogen atoms or one to three of the following radicals: halogen, C₁-C₄-alkyl, C₁-C₄-haloalkyl, C₁-C₄-alkoxy, C₁-C₄-haloalkoxy, C₃-C₆-cycloalkyl, phenyl or benzyl;
Y is oxygen, a direct bond or
for the case where Z is NOCH₃: oxygen, a direct bond or nitrogen, the nitrogen atom carrying one of the following radicals: hydrogen, C₁-C₄-alkyl or C₁-C₄-alkoxy;
Z is CHOCH₃, NOCH₃, CHCH₃ or CHCH₂CH₃;
R¹ is C₁-C₄-alkyl;
R² is C₁-C₆-alkyl, C₃-C₆-cycloalkyl, C₂-C₆-alkenyl or C₃-C₆-alkynyl, it being possible for these groups to be partly or completely halogenated and/or to carry one or two of the following radicals:
hydroxyl, cyano, isocyano, nitro, formyl, amino, formylamino, carboxyl, sulfoxyl, carbonylamino, C₁-C₈-alkylamino, di-C₁-C₈-alkylamino, C₁-C₈-alkylcarbonyl, C₁-C₈-alkoxycarbonyl, C₁-C₈-alkylaminocarbonyl, C₁-C₈-alkylsulfonyl or phenylsulfonyl;
phenyl, which can carry one to five halogen atoms and/or one to three of the following radicals:
amino, carboxyl, carbonylamino, cyano, formyl, formylamino, hydroxyl, isocyano, nitro, sulfoxyl, thiocarboxamido, thiocyanato, C₁-C₈-alkyl, C₁-C₄-haloalkyl, C₁-C₈-alkylcarbonyl, C₁-C₈-alkylcarbonyloxy, C₁-C₈-alkylcarbonylthio, C₁-C₈-alkylcarbonylamino, C₁-C₈-alkylsulfonyl, C₁-C₈-alkylsulfonyloxy, C₁-C₈-alkylsulfonylamino, C₁-C₈-alkoxy, C₁-C₄-haloalkoxy, C₁-C₈-alkoxycarbonyl, C₁-C₆-alkoxyiminoalkyl, C₁-C₈-alkylthio, C₁-C₈-alkylthiocarbonyl, C₁-C₈-alkylamino, C₁-C₈-alkylaminocarbonyl, di-C₁-C₈-alkylamino, di-C₁-C₈-alkylaminocarbonyl, C₃-C₈-cycloalkyl, C₃-C₈-cycloalkoxy, C₃-C₈-cycloalkylthio, C₃-C₈-cycloalkylamino, C₂-C₁₀-alkenyl, C₂-C₈-alkenyloxy, C₂-C₁₀-alkynyl, C₂-C₁₀-alkynyloxy, phenylsulfonyl, phenylsulfonyloxy or phenylsulfonylamino;
furyl, thienyl, pyrrolyl, pyrazolyl, imidazolyl, triazolyl, isoxazolyl, oxazolyl, oxadiazolyl, isothiazolyl, thiazolyl, thiadiazolyl, pyridyl, pyridazinyl, pyrimidyl, pyrazinyl or triazinyl, it being possible for these rings to carry one to four halogen atoms and/or one to three of the following radicals:
amino, carboxyl, carbonylamino, cyano, formyl, formylamino, hydroxyl, isocyano, nitro, sulfoxyl, thiocarboxamido, thiocyanato, C₁-C₈-alkyl, C₁-C₄-haloalkyl, C₁-C₈-alkylcarbonyl, C₁-C₈-alkylcarbonyloxy, C₁-C₈-alkylcarbonylthio, C₁-C₈-alkylcarbonylamino, C₁-C₈-alkylsulfonyl, C₁-C₈-alkylsulfonyloxy, C₁-C₈-alkylsulfonylamino, C₁-C₈-alkoxy, C₁-C₄-haloalkoxy, C₁-C₈-alkoxycarbonyl, C₁-C₆-alkoxyiminoalkyl, C₁-C₈-alkylthio, C₁-C₈-alkylthiocarbonyl, C₁-C₈-alkylamino, C₁-C₈-alkylaminocarbonyl, di-C₁-C₈-alkylamino, di-C₁-C₈-alkylaminocarbonyl, C₃-C₈-cycloalkyl, C₃-C₈-cycloalkoxy, C₃-C₈-cycloalkylthio, C₃-C₈-cycloalkylamino, C₂-C₁₀-alkenyl, C₂-C₈-alkenyloxy, C₂-C₁₀-alkynyl, C₂-C₁₀-alkynyloxy, phenylsulfonyl, phenylsulfonyloxy or phenylsulfonylamino;

2. A compound of the formula I as claimed in claim 1, where Y is oxygen and Z is CHOCH₃, NOCH₃ or CHCH₃.

3. A compound of the formula I as claimed in claim 1, where Y is NH and Z is NOCH₃.

4. A compound of the formula I as claimed in claim 2 or claim 3, where n has the value 1 and R² is A1, A1 having the meaning given in claim 1.

5. A composition against animal pests or harmful fungi, containing customary additives and an effective amount of a compound of the formula I as claimed in claim 1.

6. A composition as claimed in claim 5 for controlling animal pests from the insects, Arachnida or nematodes class.

7. A process for controlling animal pests or harmful fungi, which comprises treating the pests or harmful fungi, their environment or the plants, surfaces, materials or spaces to be kept free from them with an effective amount of a compound of the formula I as claimed in claim 1.

8. The use of the compounds I as claimed in claim 1 for preparing compositions against animal pests or harmful fungi.

9. The use of the compounds I as claimed in claim 1 for controlling animal pests or harmful fungi.

## Revendications

1. Ethers naphtyliques de formule générale I 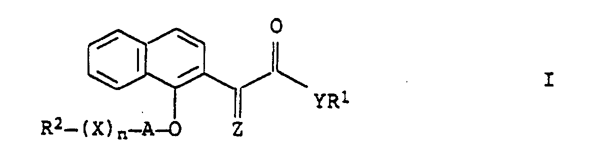 dans laquelle l'indice et les symboles ont les significations suivantes :
A : représente l'un des groupes A.1 à A.42 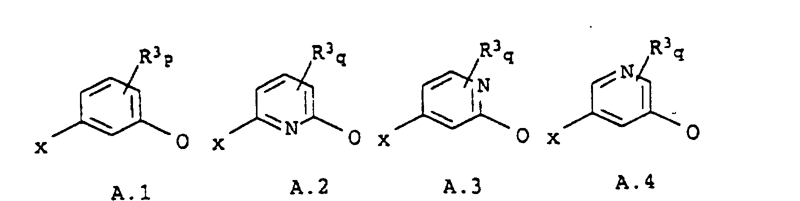 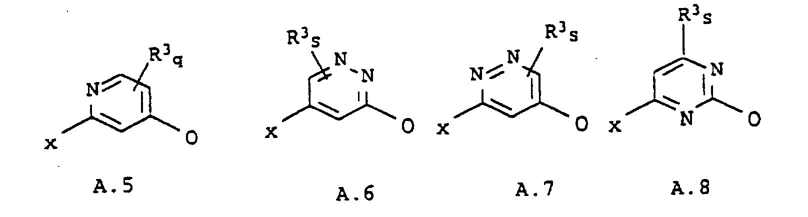 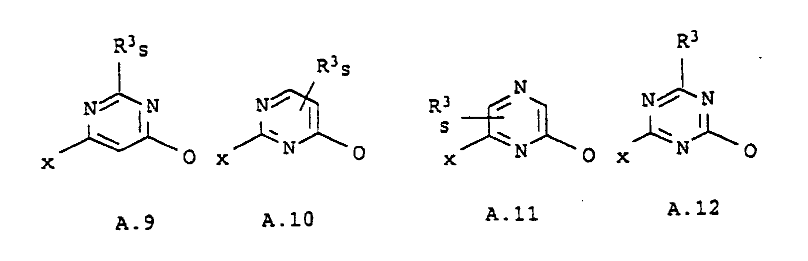 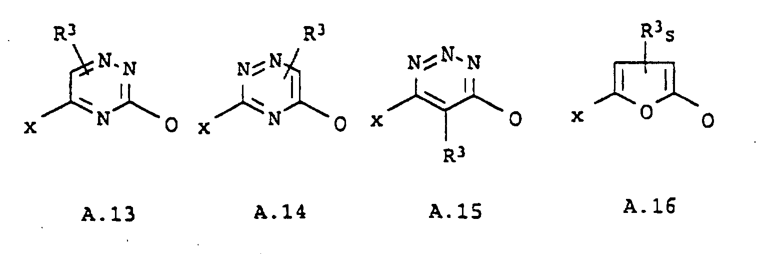 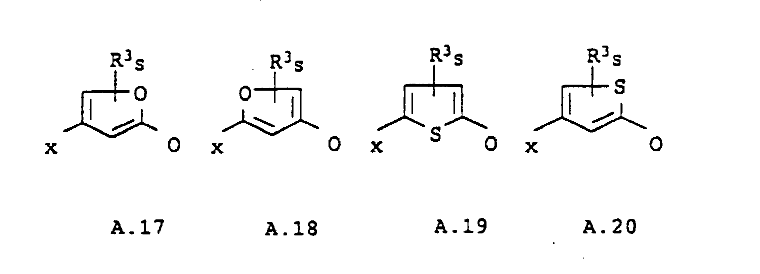 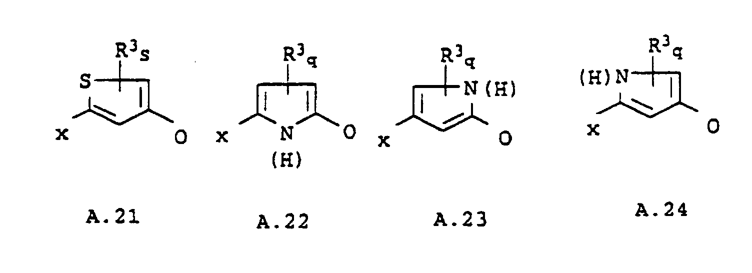 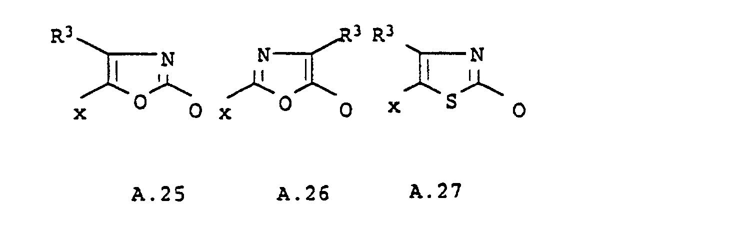 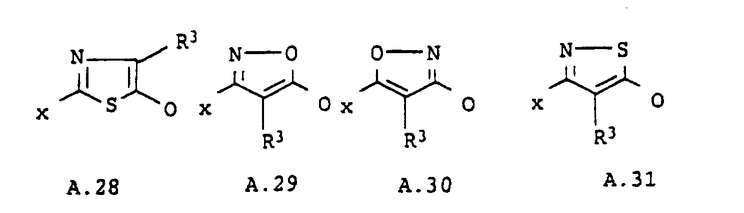 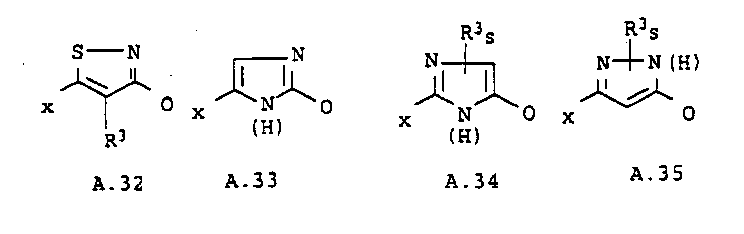 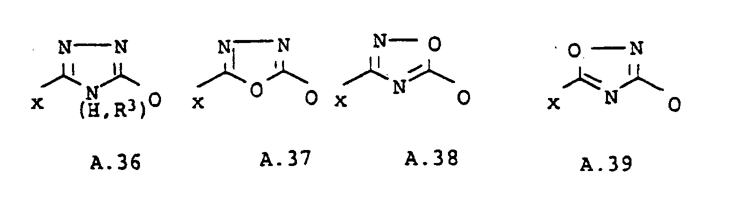 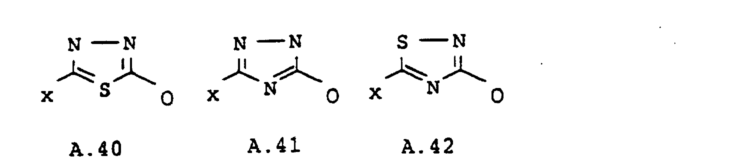 dans lesquels les signes x- et -o indiquent vers laquelle des parties de molécule voisines de A la liaison est dirigée, à savoir, vers le groupe -(X)ₙR² pour la liaison portant le signe x- et vers l'atome d'oxygène pour la liaison portant le signe -o, et
p est égal à 0, 1, 2, 3 ou 4, les substituants R³ pouvant être différents lorsque p est supérieur à 1 ;
q est égal à 0, 1, 2 ou 3, les substituants R³ pouvant être différents lorsque q est supérieur à 1 ;
s est égal à 0, 1 ou 2 et les substituants R³ peuvent être différents lorsque s est supérieur à 1 ;
les substituants R³ étant reliés à un atome de carbone du cycle ou bien, dans le cas des groupes A.22, A.23, A.24, A.33, A.34, A.35 et A.36, en outre à un atome d'azote du cycle et
R³ a les significations suivantes :
l'hydrogène, un halogène, un groupe alkyle en C1-C4, halogénoalkyle en C1-C4, halogénoalcoxy en C1-C4 et cycloalkyle en C3-C6 ;
n est égal à 0 ou 1 ;
X représente l'oxygène, le soufre ou l'azote, l'atome d'azote portant l'un des substituants suivants : l'hydrogène, un groupe alkyle en C1-C4, alcoxy en C1-C4, cycloalkyle en C3-C6, phényle ou benzyle, le cycle phényle et le cycle benzyle pouvant porter un à cinq atomes d'halogènes ou un à trois des substituants suivants : halogéno, alkyle n C1-C4, halogénoalkyle en C1-C4, alcoxy en C1-C4, halogénoalcoxy en C1-C4, cycloalkyle en C3-C6, phényle ou benzyle ;
Y représente l'oxygène, une liaison directe ou bien
dans le cas ou Z représente NOCH₃ : l'oxygène, une liaison directe ou l'azote, l'atome d'azote portant l'un des substituants suivants: l'hydrogène, un groupe alkyle en C1-C4 ou alcoxy en C1-C4 ;
Z représente CHOCH₃, NOCH₃, CHCH₃ ou CHCH₂CH₃;
R¹ représente un groupe alkyle en C1-C4
R² représente un groupe alkyle en C1-C6, cycloalkyle en C3-C6, alcényle en C2-C6 ou alcynyle en C3-C6, ces groupes pouvant être partiellement ou totalement halogénés et/ou pouvant porter un ou deux des substituants suivants :
hydroxy, cyano, isocyano, nitro, formyle, amino, formylamino, carboxyle, sulfoxyle, carbonylamino, alkylamino en C1-C8, di-(alkyle
en C1-C8)amino, (alkyle en C1-C8)carbonyle, (alcoxy en C1-C8)carbonyle, (alkyle en Cl-C8)aminocarbonyle, alkylsulfonyle en C1-C8 ou phénylsulfonyle ;
un groupe phényle qui peut porter un à cinq atomes d'halogènes et/ou un à trois des substituants suivants :
amino, carboxyle, carbonylamino, cyano, formyle, formylamino, hydroxy, isocyano, nitro, sulfoxyle, thiocarboxamido, thiocyanato, alkyle en C1-C8, halogénoalkyle en C1-C4, (alkyle en C1-C8)carbonyle, (alkyle en C1-C8)carbonyloxy, (alkyle en C1-C8)carbonylthio, (alkyle en C1-C8)carbonylamino, alkylsulfonyle en C1-C8, alkylsulfonyloxy en C1-C8, alkylsulfonylamino en C1-C8, alcoxy en C1-C8, halogénoalcoxy en C1-C4, (alcoxy en C1-C8)carbonyle, (alcoxy en C1-C6)iminoalkyle, alkylthio en C1-C8, (alkyle en C1-C8)thiocarbonyle, alkylamino en C1-C8, (alkyle en C1-C8)aminocarbonyle, di-(alkyle en C1-C8)amino, di-(alkyle en C1-C8)aminocarbonyle, cycloalkyle en C3-C8, cycloalcoxy en C3-C8, cycloalkylthio en C3-C8, cycloalkylamino en C3-C8, alcényle en C2-C10, alcényloxy en C2-C8, alcynyle en C2-C10, alcynyloxy en C2-C10, phénylsulfonyle, phénylsulfonyloxy ou phénylsulfonylamino ;
un cycle furyle, thiényle, pyrrolyle, pyrazolyle, imidazolyle, triazolyle, isoxazolyle, oxazolyle, oxadiazolyle, isothiazolyle, thiazolyle, thiadiazolyle, pyridyle, pyridazinyle, pyrimidyle, pyrazinyle ou triazinyle, ces cycles pouvant porter un à quatre atomes d'halogènes et/ou un à trois des substituants suivants :
amino, carboxyle, carbonylamino, cyano, formyle, formylamino, hydroxy, isocyano, nitro, sulfoxyle, thiocarboxamido, thiocyanato, alkyle en C1-C8, halogénoalkyle en C1-C4, (alkyle en C1-C8)carbonyle, (alkyle en C1-C8)carbonyloxy, (alkyle en C1-C8)carbonylthio, (alkyle en C1-C8)carbonylamino, alkylsulfonyle en C1-C8, alkylsulfonyloxy en C1-C8, alkylsulfonylamino en C1-C8, alcoxy en C1-C8, halogénoalcoxy en C1-C4, (alcoxy en C1-C8)carbonyle, (alcoxy en C1-C6)aminoalkyle, alkylthio en C1-C8,
(alkyle en C1-C8)thiocarbonyle, alkylamino en C1-C8, (alkyle en C1-C8)aminocarbonyle, di-(alkyle en C1-C8)amino, di-(alkyle en C1-C8)aminocarbonyle, cycloalkyle en C3-C8, cycloalcoxy en C3-C8, cycloalkylthio en C3-C8, cycloalkylamino en C3-C8, alcényle en C2-C10, alcényloxy en C2-C8, alcynyle en C2-C10, alcynyloxy en C2-C10, phénylsulfonyle, phénylsulfonyloxy ou phénylsulfonylamino ;
ou bien, dans les cas où n = 0, R² peut en outre avoir les significations suivantes : un halogène, un groupe alcoxy en C1-C6, alkylsulfoxyle en C1-C4 ou phénylsulfoxyle, le cycle phényle pouvant lui-même porter un à cinq atomes d'halogènes et/ou un à trois des substituants suivants : halogéno, alkyle en C1-C4, halogénoalkyle en C1-C4, alcoxy en C1-C4, halogénoalcoxy en C1-C4, cycloalkyle en C3-C6, phényle ou benzyle.

2. Composés de formule I de la revendication 1, dans laquelle Y représente l'oxygène et Z représente CHOCH₃, NOCH₃ ou CHCH₃.

3. Composés de formule I selon la revendication 1 dans laquelle Y représente NH et Z représente NOCH₃.

4. Composés de formule I selon la revendication 2 ou 3, dans laquelle n est égal à 1 et R² représente A1, A1 ayant les significations indiquées dans la revendication 1.

5. Produit pour combattre les parasites animaux ou les mycètes nuisibles, contenant des additifs usuels et une quantité efficace d'un composé de formule I de la revendiation 1.

6. Produit selon la revendication 5 pour la lutte contre les parasites animaux appartenant à la classe des insectes, des arachnides ou des nématodes.

7. Procédé pour combattre les parasites animaux ou les mycètes nuisibles, caractérisé par le fait que l'on traite les parasites ou mycètes nuisibles, leur habitat ou les végétaux, aires, matériaux ou locaux qu'on veut protéger contre ces parasites par une quantité efficace d'un composé de formule I de la revendication 1.

8. Utilisation des composés I de la revendication 1 pour la préparation de produits servant à combattre les parasites animaux ou les mycètes nuisibles.

9. Utilisation des composés I de la revendication 1 pour la lutte contre les parasites animaux ou les mycètes nuisibles.
